(19) Europäisches Patentamt
European Patent Office
Office européen des brevets

(11) EP 0 894 798 B1

(12) EUROPÄISCHE PATENTSCHRIFT

(45) Veröffentlichungstag und Bekanntmachung des Hinweises auf die Patenterteilung:
**09.11.2005 Patentblatt 2005/45**

(51) Int Cl.[7]: **C07D 487/04**, C08F 2/42, C08F 20/18, C08F 12/32

(21) Anmeldenummer: **98810703.3**

(22) Anmeldetag: **21.07.1998**

(54) **Polymerisierbare Diketopyrrolopyrrole**

Polymerisable diketopyrrolopyrroles

Dikétopyrrolopyrroles polymérisables

(84) Benannte Vertragsstaaten:
**CH DE FR GB IT LI**

(30) Priorität: **30.07.1997 CH 182297**

(43) Veröffentlichungstag der Anmeldung:
**03.02.1999 Patentblatt 1999/05**

(73) Patentinhaber: **Ciba Specialty Chemicals Holding Inc.**
**4057 Basel (CH)**

(72) Erfinder: **Eldin, Sameer**
**1784 Courtepin (CH)**

(56) Entgegenhaltungen:
**EP-A- 0 337 951**
**EP-A- 0 787 730**
**EP-A- 0 787 731**
**US-A- 4 585 878**

- **W.-K. CHAN ET AL.: "Rational Designs of Multifunctional Polymers" J. AMER. CHEM. SOC., Bd. 115, Nr. 25, 1993, Seiten 11735-11743, XP000652156**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 894 798 B1

Printed by Jouve, 75001 PARIS (FR)

## Beschreibung

Die vorliegende Erfindung betrifft Diketopyrrolopyrrole der Formel I

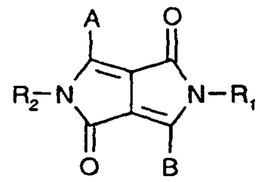

(I)

worin

$R_1$ einen Rest der Formel II

-Y-X-Z-Q (II)

bedeutet,

worin

Y für $-CR_3R_4$, $-SO_2-$, -CO-, $-Si(Hal)_2-$, -POHal- steht, worin $R_3$ und $R_4$ unabhängig voneinander Wasserstoff oder $C_1$-$C_4$-Alkyl und Hal Halogen bedeuten,

X ist eine unsubstituierte oder substituierte carbocylische oder heterocyclische Arylengruppe,

Z steht für eine Einfachbindung, ununterbrochenes lineares oder verzweigtes $C_1$-$C_{30}$-Alkylen oder ein- oder mehrfach durch ein Heteroatom, -CH=CH-, -C≡C-, -N=CH-, -CH=N- oder -NH- unterbrochenes lineares oder verzweigtes $C_2$-$C_{30}$-Alkylen, oder für $C_5$-$C_{12}$-Cycloalkylen,

Q ist -OH, -SH, $-NH_2$, Glycidyl, 1,2-Epoxiethyl,

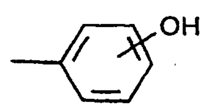

,

-CHO, -NCO, $-CH=CH_2$, $-C(CH_3)=CH_2$, $-OCO-CH=CH_2$, $-OCO-C(Me)=CH_2$, $-CH_2-CH=CH_2$, $-CH_2-OH$, $-CO-CH=CH_2$, $-CO-C(CH_3)=CH_2$, $C_5$-$C_7$-Cycloalkenyl,

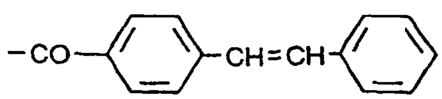 , 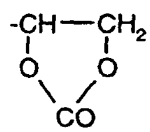 , 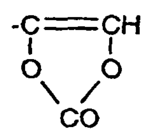 ,

$-CONHR_6$, $-COOR_6$, $-COR_6$, worin $R_6$ Wasserstoff oder $C_1$-$C_6$-Alkyl ist,

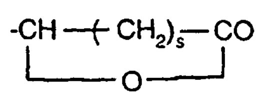 , 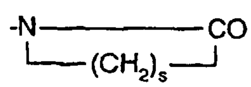

wobei s eine ganze Zahl von 1 bis 6 ist,

und

$R_2$ $C_1$-$C_6$-Alkyl,

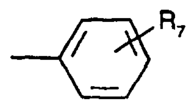

oder $R_1$ ist, und

$R_7$ Wasserstoff oder $C_1$-$C_6$-Alkyl bedeutet,

und

A und B unabhängig voneinander für eine Gruppe der Formel

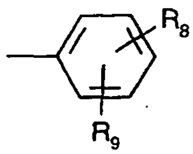 , 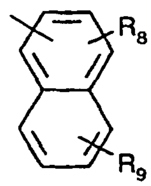 , 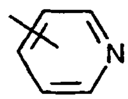 , 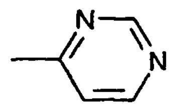 , 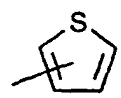 ,

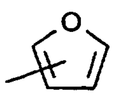 oder 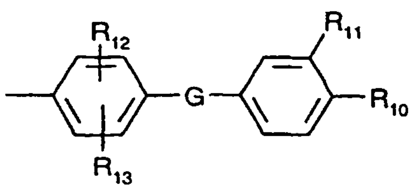

stehen, worin

$R_8$ und $R_9$ unabhängig voneinander Wasserstoff, Halogen, $C_1$-$C_{18}$-Alkyl, $C_1$-$C_{18}$-Alkoxy, $C_1$-$C_{18}$-Alkylmercapto, $C_1$-$C_{18}$-Alkylamino, $C_1$-$C_{18}$-Alkoxycarbonyl, $C_1$-$C_{18}$-Alkylaminocarbonyl, -CN, -$NO_2$, Trifluormethyl, $C_5$-$C_6$-Cycloalkyl, -CH=N-($C_1$-$C_{18}$-Alkyl),

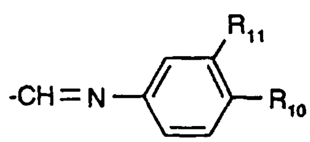 ,

Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,

G -$CH_2$-, -$CH(CH_3)$-, -$C(CH_3)_2$-, -CH=N-, -N=N-, -O-, -S-, -SO-, -$SO_2$-, -CONH- oder -$NR_7$- ist,

$R_{10}$ und $R_{11}$ unabhängig voneinander Wasserstoff, Halogen, $C_1$-$C_6$-Alkyl, $C_1$-$C_{18}$-Alkoxy oder -CN sind, $R_{12}$ und $R_{13}$ unabhängig voneinander Wasserstoff, Halogen oder $C_1$-$C_6$-Alkyl bedeuten.

Des weiteren betrifft die Erfindung ein Verfahren zur Herstellung der Diketopyrrolopyrrole I, deren Verwendung zur Herstellung von Polymeren, Polymere auf der Basis von Diketopyrrolopyrrolen I, ein Verfahren zu deren Herstellung, deren Verwendung, eingefärbte hochmolekulare organische Materialien enthaltend die erfindungsgemäßen Polymeren, ein Monomerengemisch, enthaltend die erfindungsgemäßen Diketopyrrolopyrrole I sowie die Verwendung dieses Monomerengemisches.

In EP-A 337 951 werden farbige Polymermikropartikel beschrieben, die durch Copolymerisation von zur Polymerisation befähigte reaktive Gruppen enthaltenden Pigmentderivaten und ein- oder mehrfach ethylenisch ungesättigten Verbindungen und/oder einer oder mehreren Glycidylverbindungen und/oder einem oder mehreren verschiedenen Monomeren, ausgewählt aus der Gruppe der Polyalkohole, Polycarbonsäuren, Hydroxycarbonsäuren, Lactone und Aminocarbonsäuren erhalten werden können. Aus der Menge der zahlreichen Pigmente als Ausgangsmaterial sind auch Diketopyrrolopyrrol-("DPP")-Derivate möglich, die an den beiden Stickstoffatomen jeweils eine an eine AlkylenGruppe gebundene zur Polymerisation befähigte Gruppe tragen können. Nachteilig an den in der EP-A 337 951

genannten polymeren Verbindungen ist, daß sie nicht genügend photostabil sind.

Aufgabe der vorliegenden Erfindung war es daher, Polymere auf der Basis von polymerisierbaren DPP-Derivaten zur Verfügung zu stellen, die eine verbesserte Photostabilität aufweisen. Insbesondere sollte die Stabilität gegenüber Sauerstoff und UV-Strahlung verbessert werden. Eine weitere Aufgabe bestand darin, auch Polymere und Copolymere mit verbesserten photostabilen und verarbeitungstechnischen (beispielsweise niedrigere Verarbeitungstemperatur) Eigenschaften zur Verfügung zu stellen.

Demgemäß wurden die eingangs definierten Diketopyrrolopyrrole der Formel I gefunden.

Des weiteren wurden ein Verfahren zur Herstellung der Diketopyrrolopyrrole I, deren Verwendung zur Herstellung von Polymeren, Polymere auf der Basis von Diketopyrrolopyrrolen I, ein Verfahren zu deren Herstellung, deren Verwendung, eingefärbte hochmolekulare organische Materialien enthaltend die erfindungsgemäßen Polymeren, ein Monomerengemisch, enthaltend die erfindungsgemäßen Diketopyrrolopyrrole I sowie die Verwendung dieses Monomerengemisches gefunden.

Erfindungsgemäß steht $R_1$ für einen Rest der Formel II

$$-Y-X-Z-Q \qquad \text{II}$$

dabei steht Y für $-CR_3R_4$, $-SO_2-$, -CO-, $-Si(Hal)_2-$, -POHal-, und $R_3$ und $R_4$ bedeuten unabhängig voneinander Wasserstoff oder $C_1$-$C_4$-Alkyl wie Methyl, Ethyl, n-, i-Propyl, n-, i-, sek.- oder tert.Butyl, bevorzugt Methyl, und Hal für Halogen wie Fluor, Chlor, Brom oder Iod, bevorzugt Chlor, bevorzugt sind $-CH_2-$, $-CMe_2-$, $-SO_2-$, -CO-, $-Si(Cl)_2-$, -POCl-, besonders bevorzugt $-CH_2-$, $-SO_2-$, -CO-, $-Si(Cl)_2-$, ganz besonders bevorzugt $-CH_2-$, $-SO_2-$, -CO-, insbesondere $-CH_2-$.

X ist erfindungsgemäß eine unsubstituierte oder substituierte carbocylische oder heterocyclische Arylengruppe mit bevorzugt sechs bis vierzehn Kohlenstoffatomen wie Phenylen, Naphthylen, Anthracenylen, Anthrachinonylen, Pyridinylen, Chinolinylen, bevorzugt die Gruppe

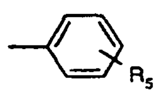

wobei $R_5$ für eine Einfachbindung in ortho-, meta- oder para-Stellung steht, oder für in ortho-, meta- oder para-Stellung stehendes -O-, bevorzugt sind para-Phenylen und para-Phenylenoxy, besonders bevorzugt para-Phenylenoxy.

Z steht für eine Einfachbindung, ununterbrochenes lineares oder verzweigtes (falls mehr als ein C-Atom vorhanden ist) $C_1$-$C_{30}$-Alkylen oder ein- oder mehrfach durch ein Heteroatom wie -O- oder -S-, bevorzugt -O-, -CH=CH-, -C≡C-, -N=CH-, -CH=N- oder -NH- unterbrochenes lineares oder verzeigtes $C_2$-$C_{30}$-Alkylen, oder $C_5$-$C_{12}$-Cycloalkylen.

$C_1$-$C_{30}$-Alkylen umfaßt sowohl die linearen wie die verzweigten Vertreter und kann beispielsweise $-CH_2-$ und $C_2$-$C_{30}$-Alkylen wie $-(CH_2)_2-$, -CH(Me)-, $-(CH_2)_3-$, $-CH_2-CH(Me)-$, $-C(Me)_2-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}$, $-(CH_2)_{21}-$, $-(CH_2)_{22}-$, $-(CH_2)_{23}-$, $-(CH_2)_{24}-$, $-(CH_2)_{25}-$,$-(CH_2)_{26}-$, $-(CH_2)_{27}-$, $-(CH_2)_{28}-$, $-(CH_2)_{29}-$, $-(CH_2)_{30}-$, bevorzugt, $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$ sowie -CH ($C_2$-$C_{30}$-Alkylen)-, oder, insbesondere falls $R_5$ für eine Einfachbindung in para-Stellung steht, bevorzugt $-CH_2-$, $-(CH_2)_2-$, und $C_{18}$-$C_{30}$-Alkylen wie $-(CH_2)_{18}-$, $-(CH_2)_{19}-$, $-(CH_2)_{20}$, $-(CH_2)_{21}-$, $-(CH_2)_{22}-$, $-(CH_2)_{23}-$, $-(CH_2)_{24}-$, $-(CH_2)_{25}-$, $-(CH_2)_{26}-$, $-(CH_2)_{27}-$, $-(CH_2)_{28}-$, $-(CH_2)_{29}-$, $-(CH_2)_{30}-$, bedeuten.

In einer bevorzugten Ausführungsform steht Z für eine Einfachbindung oder eine mehrfach mit -O- unterbrochene $C_2$-$C_{30}$-Alkylenkette wie $-CH_2-C(-)H-CH_2-O-(CH_2)p-CH_3$, wobei p für eine ganze Zahl wie 1, 2, 3 , 4 oder 5 steht, besonders bevorzugt ist $-CH_2-C(-)H-CH_2-O-(CH_2)_3-CH_3$.

Q ist -OH, -SH, $-NH_2$, Glycidyl, 1,2-Epoxiethyl

( 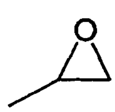 ), 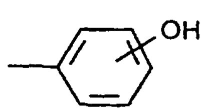 ,

-CHO, -NCO, $-CH=CH_2$, $-C(CH_3)=CH_2$, $-OCO-CH=CH_2$, $-OCO-C(Me)=CH_2$, $-CH_2-CH=CH_2$, $-CH_2-OH$, $-CO-CH=CH_2$, $-CO-C(CH_3)=CH_2$, $C_5$-$C_7$-Cycloalkenyl,

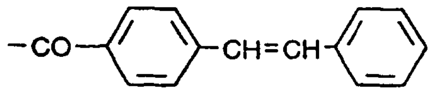 ,

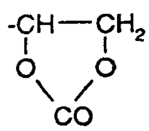 , 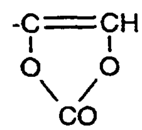 ,

$-CONHR_6$, $-COOR_6$, $-COR_6$, worin $R_6$ Wasserstoff oder $C_1$-$C_6$-Alkyl ist,

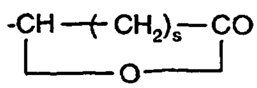 , 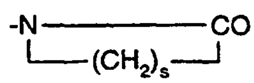

wobei s eine ganze Zahl von 1 bis 6 ist, bevorzugt steht Q für -OH, 1,2-Epoxiethyl, $-CH=CH_2$, $-C(CH_3)=CH_2$, $-CO-CH=CH_2$, $-CO-C(CH_3)=CH_2$, $-O-CO-CH=CH_2$ oder $-O-CO-C(CH_3)=CH_2$, besonders bevorzugt für -OH, 1,2-Epoxiethyl, $-CH=CH_2$, $-C(CH_3)=CH_2$, $-O-CO-CH=CH_2$ und $-O-CO-C(CH_3)=CH_2$.

$R_2$ steht erfindungsgemäß für $C_1$-$C_6$-Alkyl,

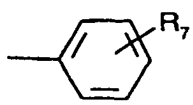 ,

wobei $R_7$ Wasserstoff oder $C_1$-$C_6$-Alkyl bedeutet, oder für $R_1$, bevorzugt für $C_1$-$C_6$-Alkyl und $R_1$, besonders bevorzugt für Methyl und $R_1$.

A und B bedeuten unabhängig voneinander eine Gruppe der Formel

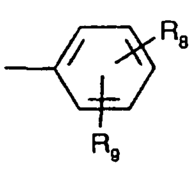 , 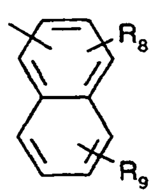 , 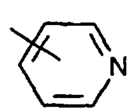 , 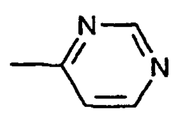 , 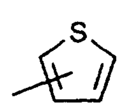 ,

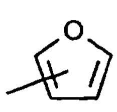 oder 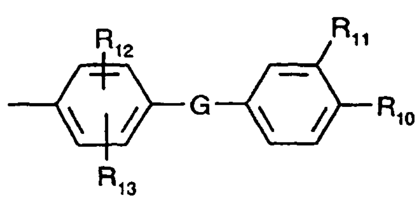

worin $R_8$ und $R_9$ unabhängig voneinander Wasserstoff, Halogen, $C_1$-$C_{18}$-Alkyl, $C_1$-$C_{18}$-Alkoxy, $C_1$-$C_{18}$-Alkylmercapto, $C_1$-$C_{18}$-Alkylamino, $C_1$-$C_{18}$-Alkoxycarbonyl, $C_1$-$C_{18}$-Alkylaminocarbonyl, -CN, $-NO_2$, Trifluormethyl, $C_5$-$C_6$-Cycloalkyl, -CH=N-($C_1$-$C_{18}$-Alkyl),

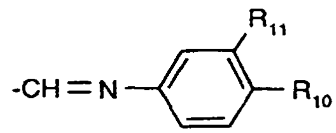 ,

Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
und G $-CH_2-$, $-CH(CH_3)-$, $-C(CH_3)_2-$, -CH=N-, -N=N-, -O-, -S-, -SO-, $-SO_2-$, -CONH- oder $-NR_7-$ ist, und
$R_{10}$ und $R_{11}$ unabhängig voneinander Wasserstoff, Halogen, $C_1$-$C_6$-Alkyl, $C_1$-$C_{18}$-Alkoxy oder -CN sind, $R_{12}$ und $R_{13}$ unabhängig voneinander Wasserstoff, Halogen oder $C_1$-$C_6$-Alkyl bedeuten.

Bevorzugt bedeuten A und B unabhängig voneinander eine Gruppe der Formel

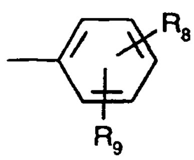 , 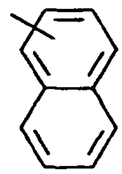 , 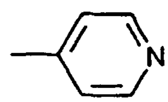 , 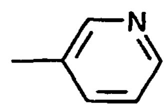

oder

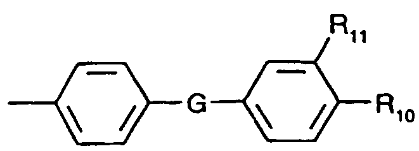 ,

worin $R_8$ und $R_9$ unabhängig voneinander Wasserstoff, Halogen wie Chlor oder Brom, $C_1$-$C_4$-Alkyl, $C_1$-$C_6$-Alkoxy, $C_1$-$C_6$-Alkylamino oder CN bedeuten,
G -O-, $-NR_7-$, -N=N- oder $-SO_2-$ ist, und
$R_{10}$ und $R_{11}$ Wasserstoff $R_7$ Wasserstoff, Methyl oder Ethyl bedeuten, besonders bevorzugt sind A und B gleich und stehen für eine Gruppe der Formel

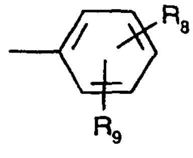

bedeuten, worin $R_8$ und $R_9$ unabhängig voneinander Wasserstoff, Methyl, tert.-Butyl, Chlor, Brom oder CN sind. $R_9$ ist bevorzugt Wasserstoff.

Halogen (oder Hal) bedeutet in der Regel Iod, Fluor, Brom oder Chlor, bevorzugt Brom oder Chlor, besonders bevorzugt Chlor.
$C_1$-$C_4$-Alkyl steht im allgemeinen für Methyl, Ethyl, n-Propyl, Isopropyl, n-Butyl, sec.-Butyl, i-Butyl, tert.-Butyl;
$C_1$-$C_6$-Alkyl steht für beispielsweise Methyl, Ethyl, n-Propyl, Isopropyl, n-Butyl, sec.-Butyl, i-Butyl, tert.-Butyl, n-Amyl,

tert.-Amyl, Hexyl;

der $C_1$-$C_{18}$-Alkyl-Rest bedeutet beispielsweise Methyl, Ethyl, n-Propyl, Isopropyl, n-Butyl, sec.-Butyl, i-Butyl, tert.-Butyl, n-Amyl, tert.-Amyl, Hexyl, Heptyl, Octyl, 2-Ethylhexyl, Nonyl, Decyl, Dodecyl, Tetradecyl, Hexadecyl oder Octadecyl;

$C_1$-$C_{18}$-Alkoxy bedeutet üblicherweise, auch in $C_1$-$C_{18}$-Alkoxycarbonyl, z.B. Methoxy, Ethoxy, n-Propoxy, Isopropoxy, Butyloxy, Hexyloxy, Decyloxy, Dodecyloxy, Hexadecyloxy oder Octadecyloxy, bevorzugt $C_1$-$C_6$-Alkoxy wie Methoxy, Ethoxy, n-Propoxy, Isopropoxy, Butyloxy, Hexyloxy;

$C_1$-$C_{18}$-Alkylmercapto steht beispielsweise für Methylmercapto, Ethylmercapto, Propylmercapto, Butylmercapto, Octylmercapto, Decylmercapto, Hexadecylmercapto oder Octadecylmercapto;

$C_1$-$C_{18}$-Alkylamino bedeutet, auch in $C_1$-$C_{18}$-Alkylaminocarbonyl, z.B. Methylamino, Ethylamino, Propylamino, Hexylamino, Decylamino, Hexadecylamino oder Octadecylamino, bevorzugt $C_1$-$C_6$-Alkylamin wie Methylamino, Ethylamino, n-Propylamino, Hexylamino.

$C_5$-$C_6$-Cycloalkyl steht z.B. für Cyclopentyl oder Cyclohexyl, insbesondere für Cyclohexyl.

$C_5$-$C_7$-Cycloalkenyl bedeutet im allgemeinen mono- oder bicyclisches Cycloalkenyl, wie Cyclopentenyl, Cyclohexenyl oder Norbornenyl.

Bevorzugte DPP-Derivate der Formel I sind solche, in denen $R_1$ für $-CH_2$-(para-Phenylen)-O-Z-Q oder -CO-(para-Phenylen)-O-Z-Q steht, und $R_2$ für Methyl oder $R_1$ steht. A, B, Z und Q stehen in dem bevorzugten Fall für die weiter oben definierten bevorzugten Reste, wobei besonders bevorzugte Reste die folgenden sind: $-CH_2$-Ph-$NH_2$, -CO-Ph-$NH_2$, $-CH_2$-Ph-SH, -CO-Ph-SH, $-CH_2$-Ph-NCO, -CO-Ph-NCO, $-SO_2$-Ph-NCO, $-CH_2$-Ph-COOH, $-CH_2$-Ph-COO-Me, $-CH_2$-Ph-CHO, $-CH_2$-Ph-$CH_2OH$, wobei Ph für 1,4-Phenylen steht. Diese Reste kann man, insbesondere falls man die Löslichkeit erhöhen möchte, mittels bekannter Maßnahmen durch Kettenverlängerung modifizieren.

Weitere bevorzugte DPP-Derivate der Formel I sind solche, in denen $R_1$ für $-CH_2$-(para-Phenylen)-Z-Q oder -CO-(para-Phenylen)-Z-Q, steht, in denen Z $C_1$-$C_{30}$-Alkylen, besonders bevorzugt $C_1$-$C_2$- und $C_{18}$-$C_{30}$-Alkylen bedeutet, und $R_2$ und Q für die weiter oben definierten Reste, vorzugsweise die bevorzugten Reste, stehen.

Ganz besonders bevorzugte DPP-Derivate I sind:

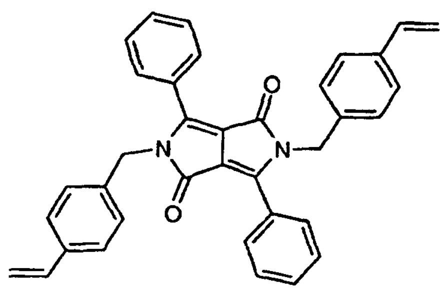

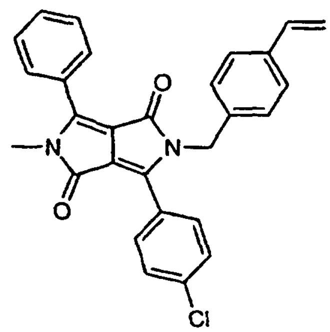

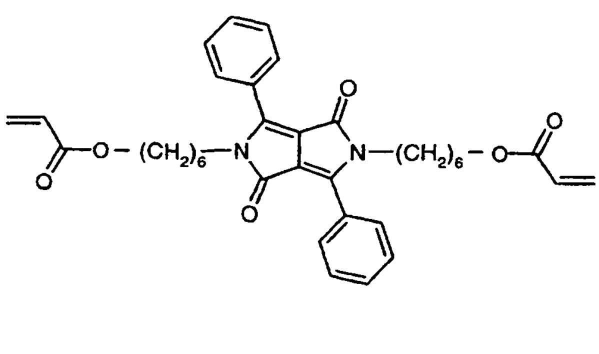

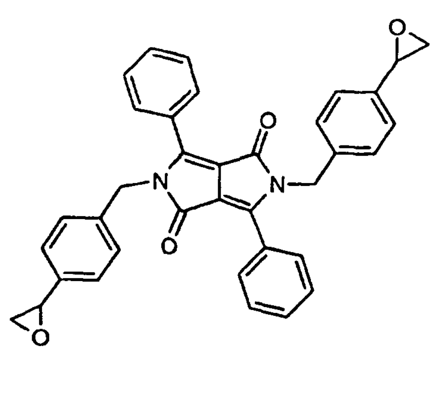

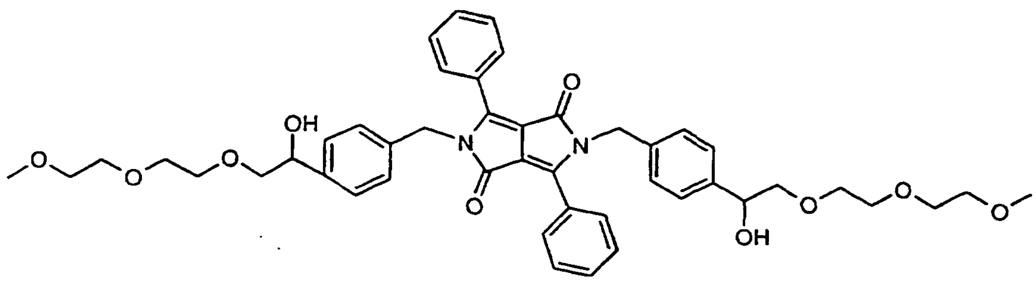

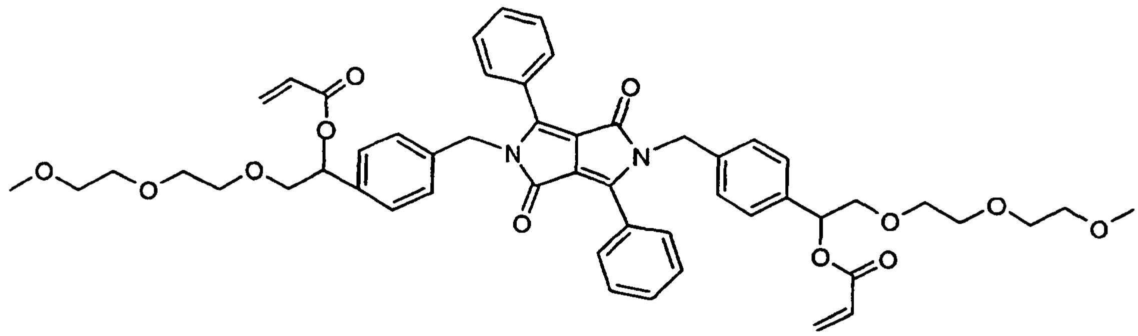

Eine weitere Ausführungsform der vorliegenden Erfindung betrifft ein Verfahren zur Herstellung der erfindungsgemäßen DPP-Derivate I, indem man bevorzugt an den N-Atomen un- oder monosubstituierte DPP-Derivate der Formel III

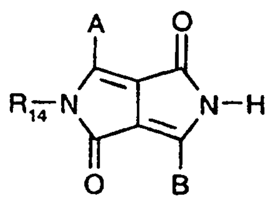

III

in der $R_{14}$ für Wasserstoff oder $R_2$ steht,
mit halogenierten Verbindungen der Formel IV

Hal-Y-X-Z-Q IV

in Gegenwart einer Base umsetzt.

Das Molverhältnis von DPP III zu Halogenverbindung IV wählt man, falls $R_{14}$ $R_2$ bedeutet, zweckmäßig im Bereich von 2:1 bis 0,9:1, vorzugsweise von 2:1 bis 1,1:1, besonders bevorzugt von 1,5:1 bis 1,3:1, und, falls $R_{14}$ für Wasserstoff steht im Bereich von 4:1 bis 1,8:1, vorzugsweise von 4:1 bis 2,2:1, besonders bevorzugt von 3:1 bis 2,6:1.

Die Verbindung DPP III kann man beispielsweise durch Umsetzung eines Pyrrolinons mit einem Nitril herstellen (siehe hierzu auch weiter unten Umsetzung von Pyrrolinon IX mit Nitril X). Insbesondere die Verbindung IIIa, in der $R_{14}$ für Wasserstoff steht, ist z.B. nach dem in der US 4,579,949 beschriebenen Verfahren erhältlich.

Als Base setzt man üblicherweise ein Alkalimetall- oder Erdalkalimetallcarbonat wie Natriumcarbonat, Kaliumcarbonat, ein Hydrid, insbesondere ein Alkalimetallhydrid wie Natriumhydrid oder Kaliumhydrid, oder ein $C_1$-$C_4$-Alkoholat ein wie die entsprechenden Natrium uind Kaliumsalze von Methanol, Ethanol, n-, i-Propanol, n-, i-, sek.- oder tert.-Butanol wie Kaliummethanolat, Natriummethanolat, Natriumethanolat, Kaliumethanolat, bevorzugt sind Alkalimetallcarbonate, insbesondere Kaliumcarbonat.

Die Menge an Base wählt man üblicherweise in Abhängigkeit der gewählten Base, beispielsweise im Bereich von 4:1 bis 1,1:1, bezogen auf das DPP III, falls $R_{14}$ für $R_2$ steht, und im Falle, daß $R_{14}$ für Wasserstoff steht, verwendet

man in der Regel die doppelte Menge an Base.

Die Reaktionstemperatur hängt in der Regel von der Art der eingesetzten Stoffe wie die Wahl der Base ab. Beispielsweise wählt man im allgemeinen eine Temperatur im Bereich von 25 bis 150°C, bei der Verwendung von Kaliumcarbonat z.B. vorzugsweise von 70 bis 150, besonders bevorzugt von 70 bis 140°C, wobei man je nach eingesetzten Edukten auch andere Temperaturbereiche wählen kann.

Bevorzugt führt man die Reaktion in einem aprotischen, polaren Lösungsmittel wie Dimethylformamid (DMF), Dimethylsulfoxid (DMSO), Dimethylacetamid (DMAC), bevorzugt DMF, durch.

Üblicherweise setzt man das Lösungsmittel, bezogen auf das DPP III, im Überschuß ein, bevorzugt in einem zehn- bis 50fachen Überschuß.

Bevorzugt fällt man das DPP I aus dem Reaktionsgemisch mit Wasser oder einem Alkohol aus und kristallisiert es um oder man engt es zur Trockne ein und kristallisiert dann um.

Des weiteren wurde gefunden, daß man die DPP-Derivate der Formel III mit der Gruppe

$$\text{Hal-Y-X-O-R}_{end}$$

in einem ersten Schritt umsetzen kann, anschließend in einem zweiten Reaktionsschritt die Gruppe -$OR_{end}$ in eine -OH Gruppe (je nach Wahl von X kann die -OH-Gruppe endständig sein oder - falls X für einen verzweigten Alkylrest, gewünschtenfalls durch Heteroatome, insbesondere -O- unterbrochen - eben nicht) umwandeln kann, und dann in einem dritten Schritt diese phenolische Endgruppe mit einem Epoxid zu den erfindungsgemäßen DPP I weiterumsetzen kann. In einer bevorzugten Ausführungsform, insbesondere wenn man die erfindungsgemäßen DPP I photochemisch polymerisieren möchte, setzt man die -OH-Gruppe(n) mit Acrylsäure- oder Methacrylsäurechlorid zu den entsprechenden Acrylaten bzw. Methacrylaten um.

Die Wahl von $R_{end}$ ist für den Erfolg der Reaktion nach bisherigen Beobachtungen nicht ausschlaggebend. Bevorzugt wählt man jedoch für $R_{end}$ eine geeignete Abgangsgruppe, besonders bevorzugt eine $C_1$-$C_4$-Alkylgruppe, insbesonders Methyl.

Als Epoxid wählt man in der Regel ein endständiges Epoxid mit einer ununterbrochenen oder ein- oder mehrfach unterbrochenen $C_3$-$C_{30}$-Alkylkette wie

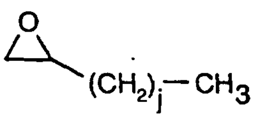

oder

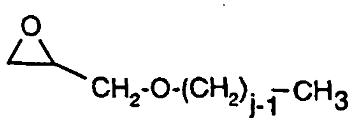

in der j eine ganze Zahl von 0 bis 27 sein kann. Beispielhaft sei der Butylglycidylether genannt.

Die Umsetzung mit der Halogenverbindung Hal-Y-X-O-$R_{end}$ nimmt man in der Regel in der analogen Weise vor wie die weiter oben bereits beschriebene Umsetzung mit dem Halogenid IV.

Die Umwandlung der -$OR_{end}$-Gruppe in die -OH-Gruppe nimmt man für gewöhnlich nach der in Tetrahedron Letters 21 (1980) 2305 beschriebenen Methode durch Umsetzung mit einem Silan wie Phenylthiotrimethylsilan vor.

In einigen Fällen kann die phenolische Gruppe auch direkt mit Hilfe von Hal-Y-X-OH eingeführt werden, beispielsweise bei Y = $CH_2$-, -$SO_2$-, -CO-, -$CMe_2$-.

Die Umsetzung eines Phenols mit einem Epoxid ist bekannt und kann beispielsweise nach der in Houben-Weyl Band 6/3, Seite 456 ff (1987) beschriebenen Methode vorgenommen werden.

Die -$CH_2$-Phenylen-Einheit kann beispielsweise durch eine entsprechende Halogenhydroxiverbindung nach der in Houben-Weyl Band 11/1, S. 42 ff (1987) beschriebenen Methode eingeführt werden. Die Ausgangsverbindung ist z.B. über eine Halogenierung einer Dihydroxiverbindung wie 1,4-Di(Hydroximethyl)phenylen, z.B. mit Sulfonylchlorid, zugänglich (siehe J.Org.Chem., S. 444-450 (1966)). Nach der Reaktion mit dem DPP III kann man die alkoholische (n) Gruppen zur Einführung von polymerisierbaren Gruppen wie die Acrylat- oder Methacrylatgruppe oder zur Kettenverlängerung verwenden, beispielsweise durch Umsetzung mit einem Epoxid (s.o.).

Die gegebenenfalls gewünschte Umsetzung des alkoholischen DPP-Derivates I mit einem Acrylsäure- oder Methacrylsäurechlorid kann man z.B. nach der in Houben-Weyl Band 8, S. 543 ff (1987) beschriebenen Methode vornehmen. In einer bevorzugten Ausführungsform verwendet man einen basischen Binder, um den freigesetzten Chlorwasserstoff oder einen Teil davon aus dem Reaktionsgemisch zu entfernen. Bevorzugte basische Binder sind beispielsweise Amine, besonders bevorzugt sekundäre Amine, ganz besonders bevorzugt ist Thiodiphenylamin (=Phenothiazin).

Falls -Z-Q für einen Oxialkylen-Rest stehen, dann lassen sich die entsprechenden DPP-Derivate I beispielsweise dadurch erhalten, daß man Epichlorhydrin mit einem Oxialkanol wie $CH_3$-($OCH_2CH_2$)k-OH (wobei k beispielsweise für 3, 4, 5, 6 oder 7 steht) zum entsprechenden Epoxid umsetzt und dann wie weiter beschrieben fortfährt. Insbesondere lange, d.h. k ≥3, insbesondere von 3≤k≤7, Oxialkylenreste verbessern im allgemeinen die Verarbeitbarkeit der erfindungsgemäßen Polymere.

Generell kann man die erfindungsgemäßen DPP I durch Kettenverlängerung modifizieren, insbesondere wenn Q für einen Rest, ausgewählt aus der Gruppe, bestehend aus -OH, 1,2-Epoxiethyl, -SH, $-NH_2$, -NCO, -COOH, $-CH_2OH$, $-CH_2-CH=CH_2$ und -CHO, steht. So kann man die -OH-, die $-CH_2OH$- und die -SH-Gruppe mit einem Epoxid zur Reaktion bringen und anschließend die neu gebildete -OH-Gruppe, falls gewünscht, mit Acrylsäure- oder Methacrylsäurechlorid zu den entsprechenden Acrylsäure- bzw. Methacrylsäurederivaten umsetzen. Den $-NH_2$-Rest kann man beispielsweise mit Isocyanatoethylmethacrylat zu $-NHCO-CH_2-CH_2-O-C(O)-C(Me)=CH_2$ umsetzen. Den NCO-Rest kann man mit beispielsweise mit Hydroxiethyl-methacrylat zu $-NHCO-CH_2-CH_2-O-C(O)-C(Me)=CH_2$ umsetzen. Die COOH-Gruppe kann man beispielsweise mit Isocyanatoethylmethacrylat zu-$COO-NHCO-CH_2-CH_2-O-C(O)-C(Me)=CH_2$ modifizieren. Eine Allylgruppe kann man z.B. mit einem Thiol wie $HS-(CH_2)_6-NH_2$ oder $HS-(CH_2)_2-O-(CH_2)_2-O-(CH_2)_2-OH$ zu $-(CH_2)_3-S-(CH_2)_6-NH_2$ oder $-(CH_2)_3-S-(CH_2)_2-O-(CH_2)_2-O-(CH_2)_2-OH$ umsetzen. Eine Aldehydgruppe, -CHO, kann man mit einem primären Amin wie $H_2N-CH_2-CH_2-O-CH_2-CH_2-OH$ zu $-CH=N-CH_2-CH_2-O-CH_2-CH_2-OH$ umsetzen. Die 1,2-Epoxiethylgruppe oder Glycidyl kann man durch Umsetzung mit einem Alkohol in an sich bekannter Art und Weise - beispielsweise durch Katalyse mit $Sn(BF_4)_2$ - derivatisieren.

Eine besondere Ausführungsform betrifft die Umsetzung des DPP-Derivates III mit einer halogenierten Verbindung IV in der oben beschrieben Art und Weise, wobei Q für die 1,2-Epoxigruppe steht. Insbesondere bevorzugt steht Hal-Y-X-Z-Q für $Cl-CH_2$-(para-Phenylen) (1,2-epoxiethyl). Auf dies Art sind Epoxisubstituierte DPP I zugänglich, die weiter - beispielsweise durch Umsetzung mit einer eine OH-Gruppe tragenden Verbindung (s.o.), derivatisiert werden können. Die daraus resultierenden OH-Gruppen tragenden DPP I können wie oben gezeigt ebenfalls weiter umgesetzt werden, insbesondere mit Acryl- oder Methacrylsäurechlorid zu den entsprechenden Acrylaten und Methacrylaten.

Die Epoxiverbindungen sind im allgemeinen nach in der Regel bekannten Methoden aus den entsprechenden eine Doppelbindung aufweisenden Verbindungen durch Umsetzung mit einer Persäure oder einem Perester, beispielsweise mit Peressigsäure, zugänglich.

Allgemein sei noch angemerkt, dass bei Öffnung eines Epoxiringes durch Umsetzung mit einem Alkohol wie oben beschrieben Isomere auftreten können, so dass die vorliegende Erfindung auch Isomerengemische mitumfasst wie die entsprechenden sekundären und primären Alkohole:

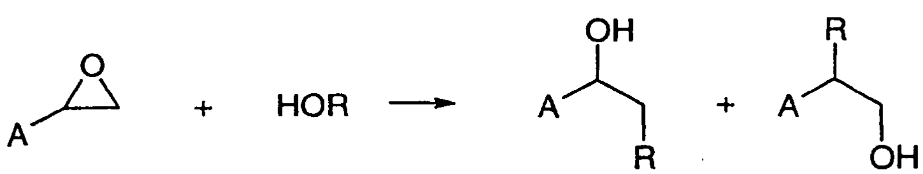

Des weiteren können auch optische Isomere, beispielsweise in Form eines Racemat-Gemisches, auftreten, die ebenfalls von der Erfindung mitumfasst werden.

Eine weitere Ausführungsform der vorliegenden Erfindung betrifft die Verwendung der Diketopyrrolopyrrole I zur Herstellung von Polymeren.

Die Polymerisation der erfindungsgemäßen DPP I nimmt man im allgemeinen in an sich bekannter Art und Weise, gewünschtenfalls in Gegenwart eines geeigneten, bevorzugt üblichen, z.B. mindestens eine Kohlenstoff-Kohlenstoff-Doppelbindung tragenden Comonomeren oder eines polyreaktionsfähige Gruppen tragenden Polymeren vor.

In einer bevorzugten Ausführungsform kann man gefärbte (Co-)Polymere herstellen, indem man eine Mischung aus erfindungsgemäßen DPP-Monomeren und weiteren, üblichen und geeigneten copolymerisierbaren Monomeren in flüssiger Phase wie in Schmelze, Lösung, Suspension und Emulsion zur Polyreaktion bringt.

Als geeignete copolymerisierbare Monomere seien beispielsweise die Gruppe der Acrylate, Methacrylate und andere übliche vinylische Monomere wie Styrol und dessen übliche Monomerderivate oder 2-N-Vinylpyrrolidon genannt. Insbesondere bevorzugte Acrylate sind monofunktionelle Acrylate wie Butandiolmonoacrylat, 2-Hydroxyethylacrylat, Butylacrylat, 2-Ethylhexylacrylat, Phenoxy-ethylacrylat, Tetrahydrofurfurylacrylat, Polypropylenglykolmonoacrylat, bifunktionelle Acrylate wie 1,6-Hexandioldiacrylat, Tripropylenglykoldiacrylat, Polyethylenglykol (200) diacrylat und Polyethylenglykol (400) diacrylat, Ethoxyliertes und propoxyliertes Neopentylglykoldiacrylat, polyfunktionelle Acrylate wie Trimethylolpropantriacrylat, Pentaerythrittriacrylat, Ethoxyliertes bzw. Propoxyliertes Trimethylolpropantriacrylat, Propoxyliertes Glyzerin-triacrylat, Tris-(2-hydroxyethyl) isocyanurattriacrylat sowie deren Mischungen untereinander. Solche Verbindungen sind beispielsweise aus "Strahlenhärtung", Curt R.Vincentzu Verlag, Hannover S. 83 bis 92 (1996) hinlänglich bekannt.

Die Herstellung dieser neuen DPP-Polymeren erfolgt üblicherweise nach allgemein bekannten Methoden, z. B. entweder durch eine Polyreaktion, d.h. durch Polymerisation (thermisch oder photochemisch), Polykondensation oder Polyaddition, oder durch eine polymeranaloge Reaktion, d.h. durch Umsetzung der erfindungsgemässen geeignete reaktive Gruppen enthaltenden DPP-Verbindungen mit bereits vorliegenden Polymeren, die selbst reaktionsfähige Gruppen aufweisen (Pfropfung). Bevorzugt ist eine photochemische Polymerisation, insbesondere wenn der Rest Q für einen Acryl- oder Methacrylrest steht.

Nach bisherigen Beobachtungen kann man mit den erfindungsgemäßen DPP-Verbindungen (DPP-Monomeren) alle bekannten Arten von Polyreaktionen durchführen. So können z.B. aus DPP-Monomeren, deren reaktive Gruppen C=C-Bindungen aufweisen, Vinyl-, Allyl-, Vinylester-, Vinylamid-, Vinylacetat- oder Vinylketon-Polymere, aus monofunktionellen DPP-Monomeren, deren reaktive Gruppen Heteroatome enthalten, Polyaldehyde, Polyisocyanate, Polyepoxide, Polyether, Polyacetone oder Polylactame, aus bifunktionellen DPP-Monomeren, deren reaktive Gruppen Heteroatome enthalten, via Polykondensation Polyester, Polyamide, Polyimide oder Polycarbonate und via Polyaddition, Polyepoxide, Polyurethane oder Polyimide hergestellt werden, wobei es sich bei der Polymerisation um radikalische, kationische oder anionische Polymerisation, Coordinationspolymerisation oder Gruppentransferpolymerisation handeln kann.

Beispiele für die Herstellung von DPP-Polymeren, ausgehend von den erfindungs-gemässen DPP-Monomeren I, sind (a) die Polymerisation zur Herstellung von DPP-Polyacrylaten oder Polymethacrylaten durch radikalische thermische Polymerisation von DPP-Acrylaten oder DPP-Methacrylaten, d.h. DPP I, in denen Q für eine Acryl- oder Methacrylgruppe steht, oder die radikalische Photopolymerisation von DPP-Acrylaten oder DPP-Methacrylaten, (b) die Polykondensation zur Herstellung von DPP-haltigen Polyestern aus DPP I-Monomeren, in denen Q für eine Hydroxigruppe steht und Disäurechloriden, oder die Herstellung von DPP-Polycarbonaten aus DPP-Diolen und Phosgen, (c) die Polyaddition zur Herstellung von DPP-Polyurethanen aus DPP-Diolen und Diisocyanaten, oder die Herstellung von DPP-Polyepoxiden aus DPP-Epoxiden und Aminen, sowie (d) die polymeranaloge Reaktion z.B. die Umsetzung eines DPP-Alkohols mit einem aus Styrol und Maleinsäureanhydrid hergestellten und demnach Anhydridgruppen aufweisenden Polymer zu einem DPP-Mono- oder -diestergruppen enthaltenden Polymer.

Gegebenenfalls enthalten die neuen DPP-Polymeren auch Additive, wie Lichtschutzmittel, Antioxidantien und UV-Absorber, die während oder nach der eigentlichen Polymerisation zugegeben werden können, z.B. auch bei der Verarbeitung der Polymeren (Extrusion). Diese Additive können auch selbst polyreaktionsfähige Gruppen aufweisen und in diesem Fall zusammen mit den DPP-Monomeren I copolymerisiert werden.

Die konkrete Durchführung zur Herstellung von Polymeren ist aus dem Stand der Technik bekannt (beispielsweise in Houben-Weyl "Methoden der Organischen Chemie", "Makromolekulare Stoffe", Band E20, Teile 1-3 (1986,1987) beschrieben).

Beispielsweise kann man, falls Q für die $-CH=CH_2-$, die Acrylat- oder Methacrylat-Gruppe steht, die Polymerisation photochemisch durchführen, wobei man dem Reaktionsgemisch üblicherweise einen der üblichen Photoinitiatoren (siehe z.B. "Chemistry & Technology of UV & EB Formulations for Coatings, Inks and Paints, Vol. 3: Photoinitiators for Free Radical and Cationic Polymerization" 1991, S. 1115-325) in einer Menge im Bereich von im allgemeinen 0,5 bis 5 Gew.-%, bezogen auf die Summe aller eingesetzten Monomeren zufügt.

Aus den erfindungsgemässen Monomeren DPP I kann man durch Einwirkung aktinischer Strahlung Formkörper aller Art, Beschichtungen und Reliefabbildungen beziehungsweise Reliefstrukturen herstellen. Die aktinische Strahlung kann von einer γ-Strahlung bis zum nahen UV-Bereich reichen. Die eingesetzte Strahlung richtet sich in der Regel im wesentlichen nach der Absorption der verwendeten Photoinitiatoren. Bevorzugt wird elektromagnetische Strahlung vom UV-Bereich bis in den sichtbaren Bereich verwendet. Geeignete Strahlenquellen sind bekannt. Es kann sich z.B um Lampen oder Laser handeln. Bevorzugt werden UV-Lampen (Quecksilberlampen) oder UV-Laser verwendet. Die Bestrahlungsdauer ist im allgemeinen u.a. von der Art der Lichtquelle abhängig; sie kann im Bereich von Stunden bis Minuten oder gar Sekunden liegen.

Besonders bevorzugt stellt man aus den erfindungsgemäßen Monomeren DPP I polymere Filme her, wobei die Schichtdicke je nach Anwendungszweck im Bereich von 5 bis 500 µm betragen kann.

Eine weitere bevorzugte Ausführungsform betrifft daher Polymere auf der Basis der Diketopyrrolopyrrole I nach Anspruch 1, erhältlich durch Polyreaktion mindestens eines Diketopyrrolopyrrols I nach Anspruch 1 oder einer Mischung bestehend aus

(A) von 0,5 bis 20, bevorzugt von 1 bis 10 Gew.-%, bezogen auf die Summe der Komponenten (A) und (B), einer Mischung bestehend aus

(a) von 100 bis 1, bevorzugt von 95 bis 20, besonders bevorzugt von 90 bis 40 Gew.-%, bezogen auf die Summe der Komponenten (a) und (b), eines Diketopyrrolopyrrols I,
(b) von 0 bis 99, bevorzugt von 5 bis 80, besonders bevorzugt von 10 bis 60 Gew.-%, bezogen auf die Summe der Komponenten (a) und (b), des Diketopyrrolopyrrols der Formel V

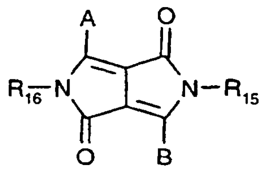

V

worin A und B die gleiche Bedeutung wie in Anspruch 1 haben,
$R_{15}$ einen eine zur Polyreaktion befähigte reaktive Gruppe aufweisenden Rest und $R_{16}$ $C_1$-$C_6$-Alkyl,

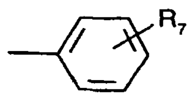

oder $R_{15}$ sind, und

(B) von 99,5 bis 80, vorzugsweise von 99 bis 90 Gew.-% , bezogen auf die Summe der Komponenten (A) und (B), eines mit den Diketopyrrolopyrrolen I und V copolymerisierbaren Monomeren,

wobei sich die Summen von (A) und (B) sowie von (a) und (b) jeweils zu 100 Gew.-% ergänzen.

Eine weitere bevorzugte Ausführungsform betrifft ein Verfahren zur Herstellung dieser erfindungsgemäßen Polymere, indem man mindestens ein Diketopyrrolopyrrol I nach Anspruch 1 oder eine Mischung bestehend aus

(A) von 0,5 bis 20, bevorzugt von 1 bis 10 Gew.-%, bezogen auf die Summe der Komponenten (A) und (B), einer Mischung bestehend aus

(a) von 100 bis 1, bevorzugt von 95 bis 20, besonders bevorzugt von 90 bis 40 Gew.-%, bezogen auf die Summe der Komponenten (a) und (b), eines Diketopyrrolopyrrols I,
(b) von 0 bis 99, bevorzugt von 5 bis 80, besonders bevorzugt von 10 bis 60 Gew.-%, bezogen auf die Summe der Komponenten (a) und (b), des Diketopyrrolopyrrols der Formel V, wie oben definiert, und

(B) von 99,5 bis 80, vorzugsweise von 99 bis 90 Gew.-% , bezogen auf die Summe der Komponenten (A) und (B), eines mit den Diketopyrrolopyrrolen I und V copolymerisierbaren Monomeren,

wobei sich die Summen von (A) und (B) sowie von (a) und (b) jeweils zu 100 Gew.-% ergänzen, zur Polyreaktion analog zu der bereits weiter oben beschrieben Polyreaktion bringt.

Eine weitere Ausführungsform betrifft Mischungen, bestehend aus

(A) von 0,5 bis 20, bevorzugt von 1 bis 10 Gew.-%, bezogen auf die Summe der Komponenten (A) und (B), einer Mischung bestehend aus

(a) von 100 bis 1, bevorzugt von 95 bis 20, besonders bevorzugt von 90 bis 40 Gew.-%, bezogen auf die Summe der Komponenten (a) und (b), eines Diketopyrrolopyrrols I,
(b) von 0 bis 99, bevorzugt von 5 bis 80, besonders bevorzugt von 10 bis 60 Gew.-%, bezogen auf die Summe der Komponenten (a) und (b), des Diketopyrrolopyrrols der Formel V, wie oben definiert, und

(B) von 99,5 bis 80, vorzugsweise von 99 bis 90 Gew.-% , bezogen auf die Summe der Komponenten (A) und (B), eines mit den Diketopyrrolopyrrolen I und V copolymerisierbaren Monomeren,

wobei sich die Summen von (A) und (B) sowie von (a) und (b) jeweils zu 100 Gew.-% ergänzen.

Unter zur Polyreaktion befähigten reaktiven Gruppen versteht man z.B. zur Polymerisation befähigte Gruppen, wie Acrylatreste, oder zur Polykondensation befähigte Gruppen, wie Hydroxi- oder Säurechloridgruppen, oder auch zur Polyaddition befähigte Gruppen, wie Epoxi-, Hydroxi- oder Isocyanatgruppen.
Vorzugsweise bedeutet $R_{15}$ einen Rest der Formel VI

$$-(CH_2)_m-CH=CH-(CH_2)_n-CH_3 \quad (VI)$$

oder

$$-(L)_t-U-(W)_v-Q \quad (VII)$$

worin m und n unabhängig voneinander eine ganze Zahl zwischen Null und 12 sind, beispielsweise 0, 1, 2, 3, 4, 5, 6, 7, 8, 9, 10, 11, 12, bevorzugt wählt man m und n so, daß die Summe m + n mindestens 4 ist, und t und v unabhängig voneinander Null oder 1 bedeuten, besonders bevorzugte Reste $R_{15}$ der Formel VII sind $-(CH_2)_n-O-CO-CH=CH_2$, mit $n \geq 4$, vorzugsweise ist n eine ganze Zahl von 4 bis 6, insbesondere 6, und $-(CH_2-CH_2-O)_m-CO-CH=CH_2$ mit m = 1, 2, 3, 4, 5, bevorzugt 2,
U ununterbrochenes oder ein- oder mehrfach durch -O- und/oder -S-, -NH-, Phenylen, -COO-, -CONH-,

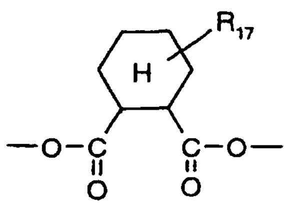

worin $R_{17}$ Wasserstoff oder Methyl ist, unterbrochenes $C_2$-$C_{18}$-Alkylen, bevorzugt $C_4$-$C_{18}$-Alkylen ist,
L

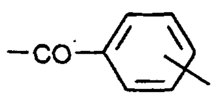 ,

$-Si(Hal)_2-$, $-Si(OC_2H_5)_2-$, $-Si(OCOCH_3)_2-$, $-CH_2-CH(OH)-$ oder - CH(CN)- und
W -O-, -NH-, -COO-, Phenylen,

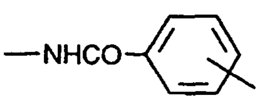 ,

bevorzugt parasubstituiert, $-Si(Hal)_2-$, $-Si(OC_2H_5)_2$ -, $-Si(OCOCH_3)_2-$, worin Hal die weiter oben angegebene Bedeutung, bevorzugt Chlor, hat,
bedeuten, und
Q die gleiche Bedeutung wie bereits oben definiert hat,
wobei die Substituenten der Verbindungen der Formel V so gewählt werden, daß Verbindungen der Formel I und der Formel V unterschiedlich sind.

Von besonderer Bedeutung sind Diketopyrrolopyrrole der Formel V, worin
$R_{15}$ einen Rest der Formel VIII

$$-U-(O)_v-Q \quad (VIII)$$

bedeutet, worin
U ununterbrochenes oder 1, 2 oder 3 Mal durch -O- und/oder einmal durch -S-, NH-,

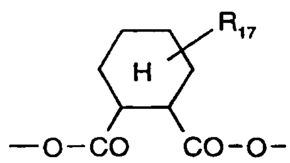

unterbrochenes $C_4$-$C_{12}$-Alkylen ist,
v und $R_{17}$ die oben angegebene Bedeutung haben, und
Q -OH; $-CH=CH_2$, $-C(CH_3)=CH_2$, $-CO-CH=CH_2$ oder $-CO-C(CH_3)=CH_2$ bedeutet.

U bedeutet vorzugsweise $-(CH_2)_q-$, wobei q eine ganze Zahl zwischen 6 und 12 wie 6, 7, 8, 9 ,10, 11 und 12 sein kann,
$-(CH_2CH_2O)_2-CH_2CH_2-$ oder

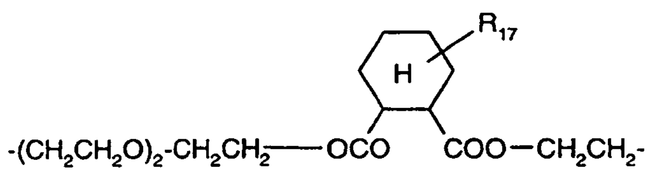 .

$R_{16}$ ist bevorzugt $CH_3$ oder $R_{15}$, wobei die gleichen Bevorzugungen, wie für $R_{15}$ gelten.

Die Diketopyrrolopyrrole der Formel V, worin $R_{15}$ einen Rest der Formel VI oder VII und $R_{16}$ $C_1$-$C_6$-Alkyl oder

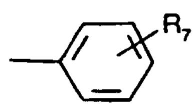

bedeuten, stellt man bevorzugt durch Umsetzung eines Pyrrolinons der Formel

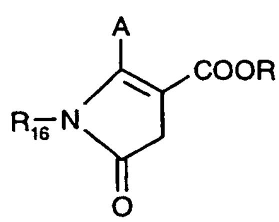 IX

worin R in der Regel so gewählt wird, daß eine übliche Abgangsgruppe ROH, bevorzugt mit R als $C_1$-$C_4$-Alkyl, gebildet wird, mit einem Nitril der Formel X, B-CN, her, wobei A und B die oben angegebene Bedeutung haben, unter Erhalt des Diketopyrrolopyrrols der Formel

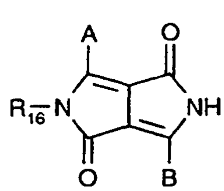

und weitere Umsetzung des letzteren mit einer Verbindung der Formel XI, $R_{15}$-Hal, oder, wenn L im Rest mit der Formel VII $-CH_2-CH(OH)-$ bedeutet, mit einer Verbindung der Formel XII

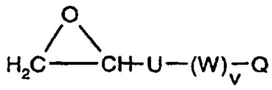

XII

worin $R_{16}$, U, W, Q und v die oben angegebene Bedeutung haben und Hal vorzugsweise Chlor oder Brom ist.

Die Reaktionsparameter wie Verhältnisse der Edukte untereinander, Temperatur etc. kann man beispielsweise analog zu dem in der US 4,778,899 beschriebenen Verfahren wählen, so daß sich nähere Angaben hierzu erübrigen.

Diketopyrrole der Formel V, worin $R_{15}$ und $R_{16}$ gleich sind, erhält man in einer bevorzugten Ausführungsform in analoger Weise ausgehend von einem Diketopyrrolopyrrol der Formel

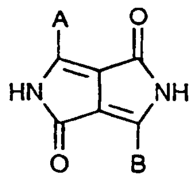

IIIa

(z.B. erhältlich nach dem in der US 4,579,949 beschriebenen Verfahren) bei Verwendung entsprechender Mengen der Verbindungen der Formeln XI oder XII.

Diketopyrrolopyrrole der Formel V, worin Q z.B. $-CO-CH=CH_2$ oder $-CO-C(CH_3)=CH_2$ und v 1 bedeuten, kann man in einer weiteren bevorzugten Ausführungsform durch Umsetzung von Diketopyrrolopyrrolen der Formel V, worin Q -OH ist, mit Acryl- bzw. Methacrylsäurechlorid hergestellt werden. In analoger Weise können z.B. auch die Gruppen $-Si(Cl)_2-CH=CH_2$,$-Si(OC_2H_5)_2-CH=CH_2$, $-Si(OCOCH_3)_2-CH=CH_2$, $-CONHR_6$, $-COOR_6$ usw. als Q bzw. W-Q eingeführt werden.

Pyrrolinone der Formel IX erhält man üblicherweise nach an sich bekannten Methoden, z.B. durch Cyclisierung einer Verbindung der Formel

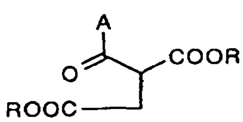

XIII

worin A und R die oben angegebene Bedeutung haben, mit einem Ammoniumsalz zum Pyrrolinon der Formel

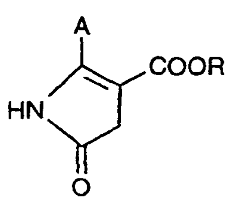

,

wie im US-Patent 4 778 899 beschrieben, und weitere Umsetzung mit einer Verbindung der Formel $R_{16}$-Hal, worin $R_{16}$ $C_1$-$C_6$-Alkyl oder

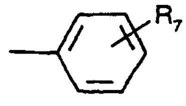

bedeutet, nach allgemein bekannten Methoden.

Die Verbindungen der Formeln X, XI, XII, IIIa und XIII sind bekannt und/oder können in Analogie zu allgemein bekannten Verfahren hergestellt werden.

Eine weitere Ausführungsform der vorliegenden Erfindung betrifft die Verwendung der erfindungsgemäßen Mischung zur Herstellung von Polymeren.

Des weiteren betrifft die vorliegende Erfindung die Verwendung der erfindungsgemäß hergestellten Polymere zur Einfärbung von hochmolekularen organischen Materialien, für Zubereitungen in der dekorativen Kosmetik, zur Herstellung von Tinten, Druckfarben, Lacken, insbesondere Automobillacken und Photolacken, photo- und elektroleitenden Polymeren, fluoreszierenden Aufhellern, Photozellen-Aggregaten, gefärbten Photoresists und Dispersionsfarben.

Die erfindungsgemäß hergestellten DPP-Polymere eignen sich insbesondere zur Einfärbung von hochmolekularen organischen Materialien wie Biopolymeren, Kunststoffen, inklusive Fasern, Gläsern, keramischen Produkten, für Zubereitungen in der dekorativen Kosmetik, zur Herstellung von Tinten, Druckfarben, Lacken, insbesondere Automobillacken und Photolacken, photo- und elektroleitenden Polymeren, fluoreszie-renden Aufhellern, Photozellen-Aggregaten, gefärbten Photoresists und Dispersionsfarben, des weiteren kann man die erfindungsgemäßen Diketopyrrolopyrrole im biomedizinischen Anwendungsbereich einsetzen wie zur Herstellung von Diagnostika sowie auf den Gebieten Impact- und NonImpact-Printing und Photo/Repro allgemein.

Beispiele geeigneter hochmolekularer organischer Materialien, die mit den erfindungsgemäßen DPP-Polymeren gefärbt werden können, sind Vinylpolymere wie Polystyrol, Poly-$\alpha$-methylstyrol, Poly-p-methylstyrol, Poly-p-hydroxystyrol, Poly-p-hydroxyphenylstyrol, Polymethylmethacrylat und Polyacrylamid sowie die entsprechenden Methacrylverbindungen, Polymethylmaleat, Polyacrylnitril, Polymethacrylnitril, Polyvinylchlorid, Polyvinyl-fluorid, Polyvinylidenchlorid, Polyvinylidenfluorid, Polyvinylacetat, Polymethylvinylether und Polybutylvinylether; von Maleinimid und/oder Maleinanhydrid abgeleitete Polymere wie Copolymere von Maleinanhydrid mit Styrol; Polyvinylpyrrolidon; ABS; ASA; Polyamide; Polyimide; Polyamidimide; Polysulfone; Polyethersulfone; Polyphenylenoxide; Polyurethane; Polyharnstoffe; Polycarbonate; Polyarylene; Polyarylensulfide; Polyepoxide; Polyolefine wie Polyethylen und Polypropylen; Polyalkadiene; Biopolymere und deren Derivate wie Cellulose, Celluloseether und -ester wie Ethylcellulose, Nitrocellulose, Celluloseacetat und Cellulosebutyrat, Stärke, Chitin, Chitosan, Gelatine, Zein; natürliche Harze; Kunstharze wie Alkydharze, Acrylharze, Phenolharze, Epoxidharze, Aminoformaldehydharze wie Harnstoff- und Melamin-Formaldehydharze; Gummi; Casein; Silikon und Silikonharze; Kautschuk, Chlorkautschuk; des weiteren Polymere, die beispielsweise als Bindemittel in Lacken verwendet werden wie Novolacke abgeleitet von $C_1$-$C_6$-Aldehyden wie Formaldehyd und Acetaldehyd und einem zweikernigen oder einkernigen, vorzugsweise einkernigen Phenol, das gewünschtenfalls mit einer oder zwei $C_1$-$C_9$-Alkylgruppen, einem oder zwei Halogenatomen oder einem Phenylring substituiert ist wie o-, m- oder p-Kresol, Xylol, p-tert.-Butylphenol, o-, m- oder p-Nonylphenol, p-Chlorphenol oder p-Phenylphenol, oder einer Verbindung mit mehr als einer phenolischen Gruppe wie Resorcin, Bis-(4-hydroxyphenyl)methan oder 2,2-Bis-(4-hydroxyphenyl)propan; sowie geeignete Mischungen der genannten Materialien.

Besonders bevorzugte hochmolekulare organische Materialien, insbesondere zur Herstellung eines Lackes, einer Druckfarbe oder Tinte, sind beispielsweise Celluloseether und -ester, wie Ethylcellulose, Nitrocellulose, Celluloseacetat und Cellulosebutyrat, natürliche Harze oder Kunstharze (Polymerisations- oder Kondensationsharze) wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, ASA, Polyphenylenoxide, Gummi, Casein, Silikon und Silikonharze sowie deren mögliche Mischungen untereinander.

Man kann auch hochmolekulare organische Materialien in gelöster Form als Filmbildner einsetzen wie Leinölfirnis, Nitrocellulose, Alkydharze, Phenolharze, Melamin- und Harnstoff/Formaldehydharze sowie Acrylharze.

Die genannten hochmolekularen organischen Verbindungen können einzeln oder in Gemischen z.B. als Granulat, plastische Massen, Schmelzen oder in Form von Lösungen, insbesondere zur Herstellung von Spinnlösungen, Lacken, Anstrichstoffen, Tinten oder Druckfarben, vorliegen.

In einer bevorzugten Ausführungsform verwendet man die erfindungsgemäßen DPP-Polymere zum Massefärben von Polyvinylchlorid, Polyamiden und insbesondere Polyolefinen wie Polyethylen und Polypropylen sowie zur Herstellung von Lacken, inklusive Pulverlacken, Tinten, Druckfarben und Anstrichfarben.

Als Beispiele für bevorzugte Bindemittel für Lacksysteme seien Alkyd/Melaminharzlacke, Acryl/Melaminharzlacke, Celluloseacetat/Cellulosebutyratlacke und Zweikomponentenlacke auf Basis mit Polyisocyanat vernetzbarer Acrylharze genannt.

Nach bisherigen Beobachtungen kann man die erfindungsgemäßen DPP-Polymere in jeder gewünschten Menge in Abhängigkeit vom Verwendungszweck dem zu färbenden Material zugeben. Beispielsweise kann man bei hochmolekularen organischen Materialien die erfindungsgemäßen DPP-Polymere in einer Menge im Bereich von 0,01 bis 40, bevorzugt von 0,1 bis 20 Gew.-%, bezogen auf das Gesamtgewicht des gefärbten hochmolekularen organischen Materials, einsetzen.

Die Einfärbung der hochmolekularen organischen Materialien mit den erfindungsgemäßen DPP-Polymeren erfolgt in der Regel dergestalt, daß man die erfindungsgemäßen DPP-Polymere, gewünschtenfalls in Form von Masterbatches, den hochmolekularen organischen Materialien unter Verwendung üblicher hierzu geeigneter Vorrichtungen wie Extruder, Walzwerke, Misch- oder Mahlapparaturen zumischt. Das so behandelte Material wird im allgemeinen

hierauf nach an sich bekannten Verfahren wie Kalandrieren, Pressen, Streichen, Gießen, Extrudieren oder Spritzgießen in die gewünschte endgültige Form gebracht.

In einer besonders bevorzugten Ausführungsform kann man photohärtbare Gruppen wie Acrylat- oder Methacrylatgruppen enthaltende DPP 1, oder auch Mischungen der oben angeführten Art von DPP I mit DPP V, in anderen photohärtbaren Monomeren lösen, was mit oder ohne Lösungsmittel geschehen kann, im letzteren Falle z.B. durch Schmelzlösen, mit entsprechenden Photoinitiatoren abmischen, und damit geeignete Substrate beschichten und die Beschichtungen mittels aktinischer Strahlung, bevorzugt UV-Strahlung, aushärten, d.h. zur Polymerisation bringen.

In einer weiteren bevorzugten Ausführungsform kann man die erfindungsgemäßen DPP-Monomeren zusammen mit anderen Monomeren, insbesondere solchen, die üblicherweise zur Herstellung der bereits weiter oben genannten Polymere eingesetzt werden, in einem Extruder zur Polyreaktion bringen (reactive extrusion, analog zu beispielsweise dem in der EP-A 337 951 beschriebenen Verfahren). So hergestellte Copolymere weisen üblicherweise die gleiche Anwendungsbreite auf wie die bislang genannten Blends aus erfindungsgemäßen DPP-Polymeren und hochmolekularen organischen Materialien.

Zur Herstellung nicht starrer Formlinge oder zur Verringerung ihrer Sprödigkeit kann man den hochmolekularen Substanzen vor der Verformung sogenannte Weichmacher zusetzen. Weichmacher können beispielsweise sein: Ester der Phosphorsäure, Phthalsäure und Sebazinsäure. Die Weichmacher können vor, während oder nach dem Einfärben der hochmolekularen Substanzen mit den erfindungsgemäßen DPP-Polymeren zugesetzt werden.

Zur Erzielung verschiedener Farbtöne kann man die erfindungsgemäßen DPP-Polymere vorteilhaft in Abmischung mit Füllstoffen, transparenten und deckenden Weiß-, Bunt- und/oder Schwarzpigmenten sowie auch herkömmlichen Glanzpigmenten in der gewünschten Menge einsetzen.

Zur Herstellung von Lacken, Anstrichstoffen, Tinten und Druckfarben dispergiert oder löst man im allgemeinen die entsprechenden hochmolekularen organischen Substanzen wie Bindemittel, Kunstharzdispersionen etc. und die erfindungsgemäßen DPP-Polymere, gewünschtenfalls zusammen mit üblichen Zusatzstoffen wie Füllmitteln, Lackhilfsmitteln, Siccativen, Weichmachern und/oder zusätzlichen Pigmenten, in einem gemeinsamen Lösungsmittel oder Lösungsmittelgemisch. Man kann dabei so verfahren, daß man die einzelnen Komponenten für sich oder auch mehrere gemeinsam dispergiert oder löst, und erst hierauf alle Komponenten zusammenbringt, oder alles auf einmal zugibt.

Bei der Applikation im Druck kann man alle industrieüblichen Druckverfahren wie Siebdruck, Tiefdruck, Bronzierdruck, Flexodruck und Offestdruck einsetzen.

Eine weitere Ausführungsform der vorliegenden Erfindung betrifft daher die durch die erfindungsgemäße Verwendung der Polymere hergestellten eingefärbten hochmolekularen organischen Materialien, Zubereitungen in der dekorativen Kosmetik, Tinten, Druckfarben, Lacken, insbesondere Automobillacke und Photolacke, photo- und elektroleitende Polymere, fluoreszierende Aufheller, Photozellen-Aggregate, gefärbte Photoresists und Dispersionsfarben, insbesondere eingefärbte hochmolekulare organische Materialien und Lacke, besonders bevorzugt Automobillacke und Photolacke.

Die erfindungsgemäß hergestellten Polymere und Copolymere auf der Basis der erfindungsgemäßen DPP-Monomere I weisen gegenüber entsprechenden Polymeren aus dem Stand der Technik eine verbesserte Photostabilität auf.

<u>Beispiele</u>

<u>Beispiel 1</u>: 14,42 g (0,05 mol) des DPP-Derivates Pigment Rot 3067E (erhältlich nach Beispiel 1 aus US 4,579,949) und 0,12 g (0,5%ig in p-Vinylbenzylchlorid ("VBC")) Hydrochinon werden in 180 ml Dimethylformamid ("DMF") unter Rühren und unter Stickstoff auf eine Temperatur im Bereich von 120-125°C erhitzt. Zur dunkelroten, breiartigen Suspension werden 20,73 g (0,150 mol) bei 250°C getrocknetes Kaliumcarbonat zugegeben (Innentemperatur = 122°C). Anschliessend werden 22,90 g (0,150 mol) VBC, verdünnt mit 20ml DMF, innerhalb von 30 Minuten dazugetropft. Die nun deutlich dünnflüssigere, dunkelrote Suspension wird nach beendeter Zugabe bei 120-125°C weitergerührt. Nach einer Reaktionszeit von 2 2/3 Stunden (ab Zugabe des VBC) wird auf Raumtemperatur gekühlt, durch ein mit Polykieselsäure (HYFLO®Supercel von Ciba Specialty Chemicals) als Filtrationshilfsmittel belegten Filter filtriert und im Vakuum bei einer Temperatur von 60°C eingeengt. Das orange Produkt fällt dabei in kleinen Klumpen aus. Das Rohprodukt, das auch wenig dunkelbraunes Oel enthält, wird mit 400 ml Ethanol unter Rückflußbedingungen vermischt, wobei sich eine sehr feine Suspension bildet. Diese wird in einem Eis/Wasser-Gemisch gekühlt, dann filtriert, anschließend der hell-orange gefärbte Filterrückstand mit kaltem Ethanol gewaschen und schliesslich im Vakuum bei 50-60°C getrocknet. Das Rohprodukt wird zweimal aus DMF umkristallisiert. Ausbeute an DPP I (mit A=B=Ph, $R_1$=$R_2$= -$CH_2$-(para-Ph)-CH=$CH_2$): 23.98 g (92.1 %), Schmelzpunkt : 120°C (polymerisiert spontan beim Schmelzen).

<u>Beispiel 2</u>: (a) Herstellung der Ausgangsverbindung

29,8 g (0,12 mol) des Pyrrolinons

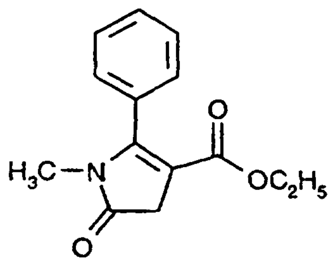

hergestellt in Anlehnung an die in Beispiel 4 von US-Patent 4,778,899 beschriebenen Methode, und 18,16 g (0,132 Mol) p-Chlorbenzonitril werden unter Stickstoffspülung vorgelegt und 180 ml 2-Methyl-1-pentanol dazugegeben. Das Gemisch wird auf 110-115°C erhitzt bis, nach etwa 30 Minuten, eine klare hellbraune Lösung entsteht. Danach werden innerhalb von zwei Stunden 12,96 g (0,24 Mol) 30%iges Natriummethylat in gesättigter Natronlauge bei einer Rührgeschwindigkeit von 510 bis 520 Umdrehungen/Minute und gleichzeitigem Abdestillieren des sich bildenden Methanol/Ethanol-Gemisches zudosiert. Es wird etwa eine Stunde bei derselben Temperatur weitergerührt und dann auf Raumtemperatur abgekühlt. Nach Zugabe von 500 ml Methanol wird mit 57,7 g (0,96 Mol) Essigsäure angesäuert. Nach kräftigem Rühren des sich bildenden dicken Breis fällt nach etwa drei Minuten das Produkt in intensiv orangeroter Farbe aus. Man verdünnt mit weiteren 100 ml Methanol und 150 ml Wasser und filtriert die orangerote Suspension. Der Filterkuchen wird mit 150 ml Methanol in 3 Portionen gewaschen und über Nacht im Vakuumtrockenschrank bei 60-70°C getrocknet. Man erhält 25,53 g (63,2 % d.Th.) des Diketopyrrolopyrrols der Formel

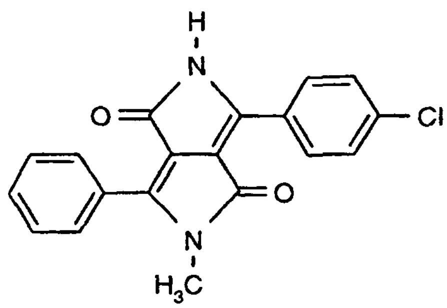

(b) 16,85 g (0,05 mol) des nach (a) hergestellten DPP-Derivates und 180 ml DMF werden unter Stickstoff auf 120-125°C erwärmt. Zur entstandenen dunkelroten Suspension werden 6,91 g (0,05 mol) bei 250°C getrocknetes Kaliumcarbonat und anschliessend 0,04 g (0,5%ig in VBC) Hydrochinon dazugegeben. Bei einer Innentemperatur von 124°C werden 11,45 g (0,075 mol) VBC, verdünnt mit 10ml DMF, innerhalb von 30 Minuten dazugetropft. Die dünnflüssige, dunkelrote Suspension wird nach beendeter Zugabe des VBC bei 120 - 125°C weitergerührt. Nach 2h 25' (ab Zugabe des VBC) ist kein DPP-Ausgangsmaterial mehr nachweisbar. Nach weiteren 20 Minuten wird die dunkelrote Suspension auf Raumtemperatur gekühlt, dann durch ein mit Polykieselsäure (siehe Beispiel 1) belegten Filter filtriert und anschliessend bei 60°C im Vakuum vollständig eingeengt. Das erhaltene dunkelrote Öl wird in 400 ml Ethanol gelöst. Die beim Abkühlen gebildete Suspension wird mit einem Eis/Wasser-Gemisch gekühlt, dann filtriert, anschließend mit kaltem Ethanol gewaschen und schließlich im Vakuum bei 40-50°C getrocknet. Das neue Produkt ist dunkelrot gefärbt und wird in Ethanol umkristallisiert. Ausbeute an DPP I (mit A=Ph, B=p-Cl-Ph, $R_2$=Me, $R_1$= $-CH_2-(p-Ph)-CH=CH_2$): 13,07 g (57,7%), Schmelzpunkt: 130°C (polymerisiert spontan beim Schmelzen),

| Elementaranalyse : | C | H | N | Cl |
|---|---|---|---|---|
| | 74,25<br>73,98 | 4,67<br>4,71 | 6,18<br>6,14 | 7,83 ber.<br>7,76 gef. |

<u>Beispiel 3</u>: Herstellung eines Polymers mittels photochemischer Polymerisation 3 g einer photohärtbaren Formulierung bestehend aus

88.8 Teilen eines handelsüblichen Acrylmonomerengemisches (ca. 80%ig in 1,6-Hexandioldiacrylat gelöst, LAROMER®EA 81, BASF),

5 Teilen des in Beispiel 1 hergestellten DPP-Monomers,

1,55 Teilen eines Photoinitiators I (ein Bis-Acyl-Phosphinoxid-Typ),
4,65 Teilen eines zweiten Photoinitiators II (IRGACURE®184, ein α-Hydroxialkylphenon von Ciba Specialty Chemicals) werden auf 188°C für zunächst 4 bis 5 Minuten erwärmt. Das Gemisch wird dann verrührt und für weitere 5 bis 6 Minuten auf 188°C erwärmt, dann wieder verrührt bis fast keine Festkörper mehr zu sehen sind, dann werden flüchtige Bestandteile im Vakuum entfernt, und anschließend nochmals für weitere 2 bis 3 Minuten auf 188°C erwärmt.

Anschließend werden mit einem 50 µm Rakel Filme auf vorbehandelte Aluminium-Platten gezogen. Diese werden alsdann mit einer Hönle-UV-Lampe (Intensität 51-53%, Abstand 19 bis 20 cm) belichtet. Die goldgelb gefärbten Filme sind nach einer Belichtungsdauer von 25 Sekunden ausgehärtet.

Beispiel 4: Die Photostabilität wird in einem ATLAS-Weatherometer Typ CI 35 getestet. Die Kenndaten für die Prüfungen :

| | |
|---|---|
| Betriebsleistung des Geräts bei 420nm | 0.92 W/m2 |
| Betriebsleistung des Xenonbrenners b. 0.92 W/m2 | 3000 W |
| Bestrahlungszyklus in h | 90.4 |
| Bestrahlungszyklus in kJ/m2 bei 420nm | 299.4 kJ/m2 |
| Bestrahlungsart | kontinuierlich |
| Schwarztafel-Temperatur | 62°C |
| Prüfraum-Temperatur | 43 +/- 3°C |
| rel. Luftfeuchtigkeit | 50 +/- 5% |

Die Photostabilität wird durch Messung der optischen Dichte nach der jeweiligen Belichtung im Weather-O-meter beurteilt und ausgedrückt in % der ursprünglichen optischen Dichte (O.D) nach x h Belichtung im Gerät.

Die Formulierung ergab nach 1000 h eine O.D. von ca. 80% und weist somit eine ganz hervorragende Photostabilität auf.

Beispiel 5: Herstellung eines DPP-Derivates der Formel V

(a) 34,60 g (0,12 mol) Diketopyrrolopyrrol der Formel III mit A = B = Phenyl und $R_{14}$ = Wasserstoff (erhältlich gemäß Beispiel 1 aus der US 4,579,949), 49,76 g (0,36 mol) Kaliumcarbonat (bei 350°C getrocknet) und 600 ml frisch destilliertes Dimethylformamid werden unter Stickstoff auf 130-135°C erhitzt. Dann werden 51,80 g (0,36 mol) 95%iges 6-Chlor-1-hexanol unter kräftigem Rühren innert etwa 10 Minuten bei dieser Temperatur zugetropft. Nach beendeter Zugabe wird die dunkelrote Suspension noch weitere 2 Stunden bei 130-135°C und dann bei Raumtemperatur über Nacht weitergerührt. Die entstandene dunkelrote Lösung mit suspendiertem Material (KCl, $K_2CO_3$) wird filtriert und der Filterkuchen wird dreimal mit 25 ml DMF gewaschen. Das Filtrat wird unter gutem Rühren zu 2 l destilliertem Wasser gegeben, wobei ein orangefarbenes Produkt ausfällt. Das Gemisch wird zum Sieden erhitzt und bei 94°C filtriert. Die im Filter zurückgebliebene klebrige rote Masse wird in 800 ml Ethanol heissgelöst, auf ca. 300 ml eingeengt und über Nacht stehen gelassen, wobei ein orangefarbiges Produkt ausfällt, das nach dem Abkühlen in Eiswasser abfiltriert und in Ethanol umkristallisiert wird. Man erhält 15,1 g (26,5% d. Th.) eines orangen kristallinen Produkt der Formel V, in der A = B = Phenyl und $R_{15}$ = $R_{16}$ = $-(CH_2)_6-OH$ (t=v=0, U=$C_6$-Alkylen, Q=-OH) bedeuten.

(b) 19,55 g (0,04 mol) des in (a) hergestellten Produktes, 0,04 g (0,2 mmol) Phenothiazin und 380 ml Dichlorethan werden unter Stickstoff zum Rückfluss geheizt. Der trüben orangefarbenen Lösung werden innerhalb etwa 30 Minuten unter Rückfluss (80°C) und Rühren 14,48 g (0,16 mol) Acrylsäurechlorid zugetropft. Nach beendeter Zugabe wird mit 20 ml Dichlorethan nachgespült. Die gelborange Lösung wird 7 Stunden am Rückfluss gerührt, anschliessend auf Raumtemperatur und über Nacht stehen gelassen. Die orangerote, ganz leicht trübe Lösung wird im Scheidetrichter fünfmal mit 100 ml 5%iger Natronlauge und 2 Mal mit deionisiertem Wasser gewaschen und mit $MgSO_4 \cdot H_2O$ getrocknet und filtriert. Das orangerote klare Filtrat wird im Rotationsverdampfer vollständig eingeengt. Das zurückgebliebene rote Öl erstarrt über Nacht zu einer festen Masse, die aus Ethanol umkristallisiert wird. Man erhält 23,1 g (96,7% d.Th.) eines kristallinen Produktes der Formel

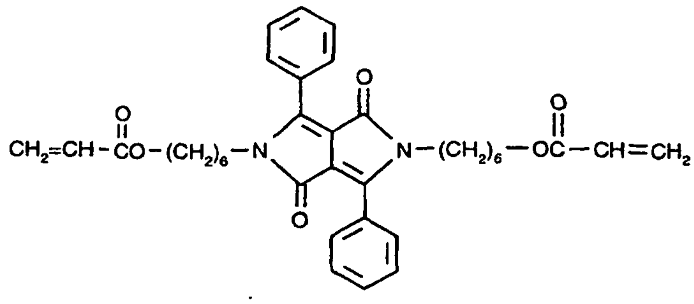

mit Schmelzpunkt 79,7-80,1 °C.

Beispiel 6: Es werden jeweils 5 g der Formulierungen hergestellt. Die Komponenten werden in einem Reagenzglas vorgelegt und mit einem Glasstab verrührt, dann bei einer Temperatur von 140°C (Mischungen) erwärmt bis durch Rühren eine klare, orange-rote, viskose Lösung entsteht. Die Filme werden mit einer 50µm Rakel auf vorbehandelte Alu-Platten gezogen. Die Belichtung erfolgt mit einer Hönle UV-Lampe. Bedingungen: Leistung ca. 92.5 mW/$cm^2$, Intensität 50-52%, Abstand 19-20 cm. Die untenstehende Tabelle gibt die Mengen der Ausgangsstoffe sowie einige Reaktionsparameter wieder.

Tabelle:

| Formulierung | 1 | 2 | 3 | 4 | 5 | 6 | 7 | 8 | 9 | 10 | 11 |
|---|---|---|---|---|---|---|---|---|---|---|---|
| Bsp.5 | 10% | 9% | 8% | 7% | 6% | 5% | 4% | 3% | 2% | - | - |
| Bsp.1 | - | 1% | 2% | 3% | 4% | 5% | 6% | 7% | 8% | 10% | 4% |
| Monomer I | 83.8% | 83.8% | 83.8% | 83.8% | 83.8% | 83.8% | 83.8% | 83.8% | 83.8% | 83.8% | 89.8% |
| PI I | 2.5% | 2.5% | 2.5% | 2.5% | 2.5% | 2.5% | 2.5% | 2.5% | 2.5% | 2.5% | 2.5% |
| PI II | 3.7% | 3.7% | 3.7% | 3.7% | 3.7% | 3.7% | 3.7% | 3.7% | 3.7% | 3.7% | 3.7% |
| Verarbeitungstemp. | 80°C | 140°C | 140°C | 140°C | 140°C | 140°C | 140°C | 140°C | 140°C | ca. 190°C | 150°C |
| Gebrauchsdauer | > 2h | | 60 min | | 60 min | | 50 min | | 50 min | ≤ 15' | |
| Photohärtungszeit | 7' | | 5.5' | | 5' | | 4.5' | | 4' | | 0.5' |
| Farbdifferenz $\Delta E^*_{ab}$ | | | | | | | | | 2.6 | | 1.8 |

Als Monomer I wird LAROMER®EA 81 (siehe Beispiel 3) eingesetzt;
PI I bedeutet Photoinitiator I, es wird der in Beispiel 3 verwendete Photoinitiator I eingesetzt;
PI II bedeutet Photoinitiator II, es wird der in Beispiel 3 verwendete Photoinitiator II eingesetzt.

Die Farbdifferenz $\Delta E^*_{ab}$ wird in einem Weather-O-meter bestimmt, wobei man zunächst vor der Bestrahlung die Farbwerte $L^*_1$, $a^*_1$ und $b^*_1$ des CIE-Farbraumes (Commission Internationale de l'Eclairage) bestimmt, anschliessend 1000 Stunden bestrahlt und danach die geänderten Farbwerte $L^*_2$, $a^*_2$ und $b^*_2$ misst. Die Farbdifferenz $\Delta E^*_{ab}$ erhält man aus der Beziehung

$$\Delta E^*_{ab} = \sqrt{(\Delta L^*)^2 + (\Delta a^*)^2 + (\Delta b^*)^2}$$

wobei $\Delta L^* = L^*_2 - L^*_1$; $\Delta a^* = a^*_2 - a^*_1$; $\Delta b^* = b^*_2 - b^*_1$

Die Gebrauchsdauer ist die Zeit, während der die jeweilige Mischung verarbeitet werden kann, bevor es zu einer (vorzeitigen) Polymerisation kommt.

Wie ersichtlich weisen die Mischungen eine hervorragende Verarbeitungscharakteristik und gleichzeitig eine sehr kurze Photohärtungszeit auf. Des weiteren ist die Farbdifferenz $\Delta E^*_{ab}$ ein Mass für die Photostabilität. Danach ist die Photostabilität umso besser, je geringer die Farbdifferenz $\Delta E^*_{ab}$ ist. Die oben gefundenen Farbdifferenzen (Formulierungen 9 und 11) sind sehr gute Werte.

Die folgenden Beispiele werden alle unter einer Stickstoff-Schutzgasatmosphäre ausgeführt:

Beispiel 7: Zu einer auf 40°C erhitzten Mischung, bestehend aus 91,6 g VBC, 16,4 g Natriumacetat und 400 ml Essigsäureethylester, werden 171,1 g einer 40%igen Peressigsäure-Lösung während 40 min zugetropft. Dabei wird Reaktionstemperatur mit Hilfe eines Wasserbades so gesteuert, dass sie 45°C nicht übersteigt. Der Reaktionsablauf wird gaschromatographisch kontrolliert. Nach 3h wird die Reaktion (Produkt-Gehalt 70-75%) abgebrochen und sofort aufgearbeitet. Zur Aufarbeitung wird die Reaktionslösung solange mit einer 10%igen Natriumhydrogencarbonat-Lösung (insgesamt ca. 1 Liter) ausgeschüttelt bis keine starke Schaumentwicklung mehr feststellbar ist. Die organischen Phasen werden über Magnesiumsulfat getrocknet, dann filtriert und das Filtrat an einem Rotationsverdampfer eingeengt. Das Rohprodukt wird durch eine Hochvakuum-Destillation gereinigt.
Ausbeute: 50% (nach Destillation), Reinheit (GC): 99%.

Beispiel 8: Zu einer auf 80°C erhitzten Suspension von 28,8 g eines käuflichen DPP-Pigmentes (ausgehend von Formel I sind dabei A = B = Phenyl und $R_1 = R_2 = H$) und 750 ml DMF fügt man 41,5 g Kaliumcarbonat hinzu und lässt 5 min rühren. Danach tropft man während 5 min 50,6 g der Epoxiverbindung aus Beispiel 7 hinzu. Die Reaktionsmischung lässt man 4 h bei 80°C rühren und filtriert dann bei dieser Temperatur über einen mit einem Filtrationshilfsmittel (siehe Beispiel 1) belegten Filter. Danach wird das Filtrat unter Hochvakuum eingeengt. Zu dem breiartigen Rückstand fügt man unter Rühren 900 ml Essigsäureethylester hinzu und lässt die erhaltene Suspension über Nacht stehen. Nach Filtration wird im Vakuum-Ofen bei 60°C getrocknet.
Ausbeute: 39,0 g (69% d. Th); Schmp.: 188°C

Beispiel 9: Eine Mischung aus 19,3 g des in Beispiel 8 hergestellten Epoxi-substituierten DPP-Derivates, 420 g Diethylenglykolmonomethylether und 0,3 ml einer 50%igen wässrigen $Sn(BF_4)_2$-Lösung wird während einer Stunde auf 110°C erhitzt. Danach wird die erhaltene Lösung in einer Atmosphäre unter reduziertem Druck eingeengt, und die eingeengte Lösung in 100 ml Ethanol gelöst und anschliessend in Eiswasser ausgefällt. Dann filtriert man und kristallisiert den Filterrückstand in Ethanol.
Ausbeute: 19,2 g (70%). Schmp.: 85-90°C

Beispiel 10: 15,8 g des in Beispiel 9 erhaltenen Produktes, 10 mg Thiodiphenylamin und 100 ml Dichlorethan werden auf eine Temperatur im Bereich von 80-85°C erhitzt. Dann werden während einer Stunde 7,24 g Acrylsäurechlorid zu dieser Mischung getropft. Nach 4,5 h Rühren bei 80-85°C wird die Reaktion durch Abkühlen auf Raumtemperatur beendet. Anschliessend schüttelt man die so erhaltene Mischung im Scheidetrichter mit je 3 x 100 ml 1 N NaOH und je 3 x 100 ml deionisiertem Wasser aus. Die vereinigten organischen Phasen werden über Magnesiumsulfat getrocknet, dann filtriert und das Filtrat in einem Rotationsverdampfer vollständig eingeengt. Der ölige Rückstand wird in 100 ml Ethanol gelöst, die ethanolische Lösung in Eiswasser während zwei Stunden abgekühlt und dann filtriert. Der Filterrückstand wird mit kaltem Ethanol gewaschen und in einer Atmosphäre unter vermindertem Druck bei Raumtemperatur getrocknet. Ausbeute: 16,5 g (92%);
Schmp.: 75 - 80°C.

## Patentansprüche

1. Diketopyrrolopyrrole der Formel I

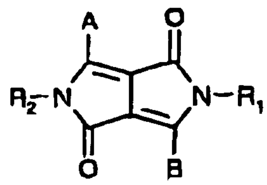

(I)

worin

$R_1$ einen Rest der Formel II

-Y-X-Z-Q (II)

bedeutet,

worin

Y für $-CR_3R_4$, $-SO_2-$, -CO-, $-Si(Hal)_2-$, -POHal- steht, worin $R_3$ und $R_4$ unabhängig voneinander Wasserstoff oder $C_1$-$C_4$-Alkyl und Hal Halogen bedeuten,

X ist ein Phenylen-, Naphthylen-, Anthracenylen-, Anthrachinonylen-, Pyridinylen-, Chinolinylen- oder Phenylenoxyrest,

Z steht für eine Einfachbindung, ununterbrochenes lineares oder verzweigtes $C_1$-$C_{30}$-Alkylen oder ein- oder mehrfach durch -O-, -S-, -CH=CH-, -C≡C-, -N=CH-, -CH=N- oder -NH- unterbrochenes lineares oder verzweigtes $C_2$-$C_{30}$-Alkylen, oder für $C_5$-$C_{12}$-Cycloalkylen,

Q ist -OH, -SH, $-NH_2$, Glycidyl, 1,2-Epoxiethyl.

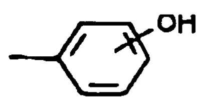 ,

-CHO, -NCO, $-CH=CH_2$, $-C(CH_3)=CH_2$, $-OCO-CH=CH_2$, $-OCO-C(Me)=CH_2$, $-CH_2-CH=CH_2$, $-CH_2-OH$, $-CO-CH=-CH_2$, $-CO-C(CH_3)=CH_2$, $C_6$-$C_7$-Cycloalkenyl,

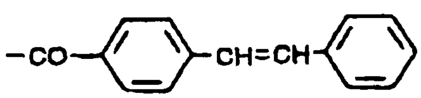 , 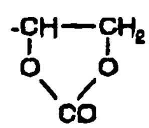 , 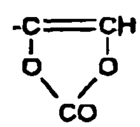 ,

$-CONHR_8$, $-COOR_6$, $-COR_6$, worin $R_6$ Wasserstoff oder $C_1$-$C_6$-Alkyl ist,

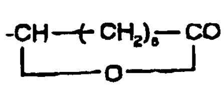 , 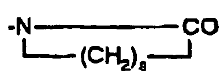

wobei s eine ganze Zahl von 1 bis 6 ist,

und

$R_2$ $C_1$-$C_6$-Alkyl,

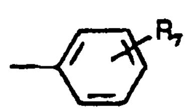

oder $R_1$ ist, und

$R_7$ Wasserstoff oder $C_1$-$C_6$-Alkyl bedeutet,

und
A und B unabhängig voneinander für eine Gruppe der Formel

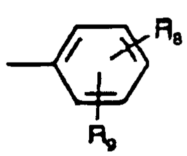 , 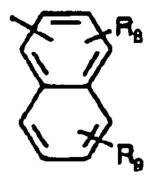 , 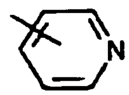 , 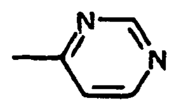 , 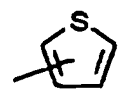 ,

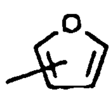 oder 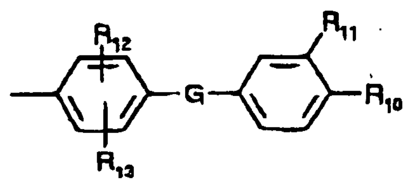

stehen, worin

$R_8$ und $R_9$ unabhängig voneinander Wasserstoff, Halogen, $C_1$-$C_{18}$-Alkyl, $C_1$-$C_{18}$-Alkoxy, $C_1$-$C_{18}$-Alkylmercapto, $C_1$-$C_{18}$-Alkylamino, $C_1$-$C_{18}$-Alkoxycarbonyl, $C_1$-$C_{18}$-Alkylaminocarbonyl, -CN, -$NO_2$, Trifluormethyl, $C_5$-$C_8$-Cycloalkyl, -CH=N-($C_1$-$C_{18}$-Alkyl),

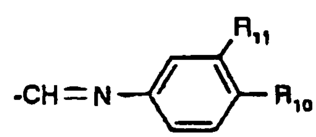 ,

Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,

G -$CH_2$-, -$CH(CH_3)$-, -$C(CH_2)_2$-, -CH=N-, -N=N-, -O-, -S-, -SO-, -$SO_2$-, -CONH- oder -$NR_7$- ist,

$R_{10}$ und $R_{11}$ unabhängig voneinander Wasserstoff, Halogen, $C_1$-$C_8$Alkyl, $C_1$-$C_{18}$-Alkoxy oder -CN sind, $R_{12}$ und $R_{13}$ unabhängig voneinander Wasserstoff, Halogen oder $C_1$-$C_6$-Alkyl bedeuten.

**2.** Verfahren zur Herstellung der Diketopyrrolopyrrole I nach Anspruch 1, **dadurch gekennzeichnet, daß** man DPP-Derivate der Formel III

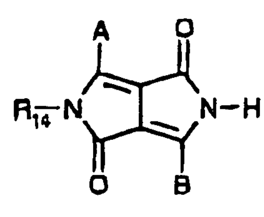

III

in der $R_{14}$ für Wasserstoff oder $R_2$ steht,
mit halogenierten Verbindungen der Formel IV

$$\text{Hal-Y-X-Z-Q} \qquad \text{IV}$$

worin Hal, Y, X, Z und Q die in Anspruch 1 angegebene Bedeutung haben, in Gegenwart einer Base zur Reaktion bringt.

**3.** Verwendung der Diketopyrrolopyrrole I nach Anspruch 1 oder hergestellt nach Anspruch 2 zur Herstellung von Polymeren.

**4.** Polymere auf der Basis der Diketopyrrolopyrrole I nach Anspruch 1, erhältlich durch Polyreaktion mindestens eines Diketopyrrolopyrrols I nach Anspruch 1 oder einer Mischung bestehend aus

(A) von 0,5 bis 20, bevorzugt von 1 bis 10 Gew.-%, bezogen auf die Summe der Komponenten (A) und (B), einer Mischung bestehend aus

(a) von 100 bis 1, bevorzugt von 95 bis 20, besonders bevorzugt von 90 bis 40 Gew.-%, bezogen auf die Summe der Komponenten (a) und (b), eines Diketopyrrolopyrrols I,
(b) von 0 bis 99, bevorzugt von 5 bis 80, besonders bevorzugt von 10 bis 60 Gew.-%, bezogen auf die Summe der Komponenten (a) und (b), des Diketopyrrolopyrrols der Formel V,

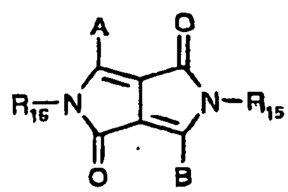

**V**

worin A und B die gleiche Bedeutung wie in Anspruch 1 haben,
$R_{15}$ einen eine zur Polyreaktion befähigte reaktive Gruppe aufweisenden Rest und $R_{16}C_1$-$C_6$-Alkyl,

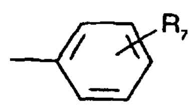

oder $R_{15}$ sind,

(B) von 99,5 bis 80, vorzugsweise von 99 bis 90 Gew.-% , bezogen auf die Summe der Komponenten (A) und (B), eines mit den Diketopyrrolopyrrolen I und V copolymerisierbaren Monomeren,

wobei sich die Summen von (A) und (B) sowie von (a) und (b) jeweils zu 100 Gew.-% ergänzen.

**5.** Verfahren zur Herstellung der Polymeren nach Anspruch 4, **dadurch gekennzeichnet, daß** man mindestens ein Diketopyrrolopyrrol I nach Anspruch 1 oder eine Mischung bestehend aus

(A) von 0,5 bis 20, bevorzugt von 1 bis 10 Gew.-%, bezogen auf die Summe der Komponenten (A) und (B), einer Mischung, bestehend aus

(a) von 100 bis 1, bevorzugt von 95 bis 20, besonders bevorzugt von 90 bis 40 Gew.-%, bezogen auf die Summe der Komponenten (a) und (b), eines Diketopyrrolopyrrols I,
(b) von 0 bis 99, bevorzugt von 5 bis 80, besonders bevorzugt von 10 bis 60 Gew.-%, bezogen auf die Summe der Komponenten (a) und (b), des Diketopyrrolopyrrols der Formel V nach Anspruch 4, und

(B) von 99,5 bis 80, vorzugsweise von 99 bis 90 Gew.-% , bezogen auf die Summe der Komponenten (A) und (B), eines mit den Diketopyrrolopyrrolen I und V copolymerisierbaren Monomeren,

wobei sich die Summen von (A) und (B) sowie von (a) und (b) jeweils zu 100 Gew.-% ergänzen, zur Polyreaktion bringt.

**6.** Verwendung der Polymere nach Anspruch 4 oder hergestellt nach Anspruch 5 zur Einfärbung von hochmolekularen organischen Materialien, für Zubereitungen in der dekorativen Kosmetik, zur Herstellung von Tinten, Druckfarben, Lacken, insbesondere Automobillacken und Photolacken, photo- und elektroleitenden Polymeren, fluoreszierenden Aufhellern, Photozellen-Aggregaten, gefärbten Photoresists und Dispersionsfarben.

**7.** Eingefärbte hochmolekulare organische Materialien und Lacke erhältlich durch die Verwendung von Anspruch 6.

**8.** Mischung bestehend aus

(A) von 0,5 bis 20, bevorzugt von 1 bis 10 Gew.-%, bezogen auf die Summe der Komponenten (A) und (B), einer Mischung bestehend aus

(a) von 100 bis 1, bevorzugt von 95 bis 20, besonders bevorzugt von 90 bis 40 Gew.-%, bezogen auf die Summe der Komponenten (a) und (b), eines Diketopyrrolopyrrols I,
(b) von 0 bis 99, bevorzugt von 5 bis 80, besonders bevorzugt von 10 bis 60 Gew.-%, bezogen auf die Summe der Komponenten (a) und (b), des Diketopyrrolopyrrols der Formel V nach Anspruch 4, und

(B) von 99,5 bis 80, vorzugsweise von 99 bis 90 Gew.-% , bezogen auf die Summe der Komponenten (A) und (B), eines mit den Diketopyrrolopyrrolen I und V copolymerisierbaren Monomeren,

wobei sich die Summen von (A) und (B) sowie von (a) und (b) jeweils zu 100 Gew.-% ergänzen.

**9.** Verwendung der Mischung nach Anspruch 8 zur Herstellung von Polymeren.

**10.** Diketopyrrolopyrrol der Formel

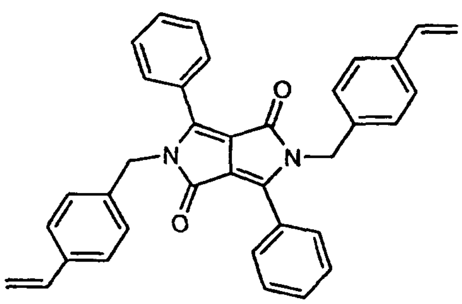

## Claims

**1.** A diketopyrrolopyrrole of formula 1

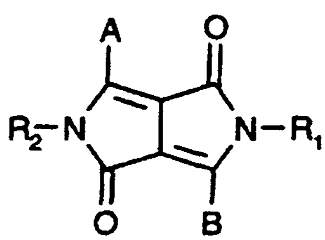

(I),

wherein
$R_1$ is a radical of formula II

**-Y-X-Z-Q (II),**

wherein

Y is $-CR_3R_4$, $-SO_2-$, -CO-, $-Si(Hal)_2$, or -POHal-, wherein $R_3$ and $R_4$ are each independently of the other hydrogen or $C_1$-$C_4$alkyl, and Hal is halogen,

X is a phenylene, naphthylene, anthracenylene, anthraquinonylene, pyridinylene, quinolinylene or phenyleneoxy radical,

Z is a single bond, uninterrupted linear or branched $C_1$-$C_{30}$alkylene, or linear or branched $C_2$-$C_{30}$alkylene which is interrupted once or more than once by -O-, -S-, -CH=CH-, -C=C-, -N=CH-, -CH=N- or -NH-, or is $C_5$-$C_{12}$cycloalkylene,

Q is -OH, -SH, $-NH_2$, glycidyl, 1,2-epoxyethyl,

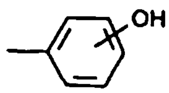 ,

-CHO, -NCO, $-CH=CH_2$, $-C(CH_3)=CH_2$, $-OCO-CH=CH_2$, $-OCO-C(Me)=CH_2$, $-CH_2-CH=CH_2$, $-CH_2-OH$, $-CO-CH=CH_2$, $-CO-C(CH_3)=CH_2$, $C_5$-$C_7$cycloalkenyl,

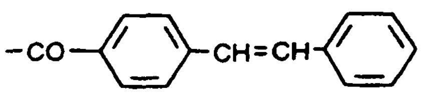 , 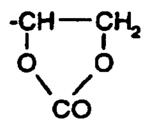 , 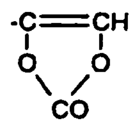 ,

$-CONHR_6$, $-COOR_6$, $-COR_6$, wherein $R_6$ is hydrogen or $C_1$-$C_6$alkyl,

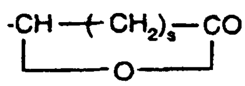 , 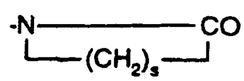

wherein s is an integer from 1 to 6, and

$R_2$ is $C_1$-$C_6$alkyl,

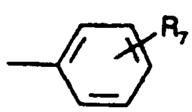

or $R_1$, and

$R_7$ is hydrogen or $C_1$-$C_6$alkyl,

and

A and B are each independently of the other a group of formula

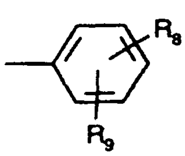 , 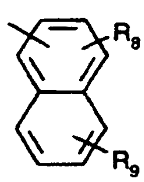 , 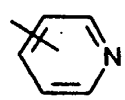 , 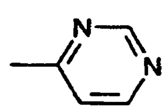 , 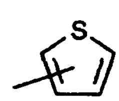 ,

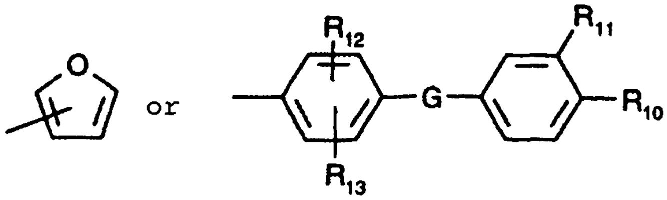

wherein
$R_8$ and $R_9$ are each independently of the other hydrogen, halogen, $C_1$-$C_{18}$alkyl, $C_1$-$C_{18}$alkoxy, $C_1$-$C_{18}$alkylmercapto, $C_1$-$C_{18}$alkylamino, $C_1$-$C_{18}$alkoxycarbonyl, $C_1$-$C_{18}$alkylaminocarbonyl, -CN, -$NO_2$, trifluoromethyl, $C_5$-$C_6$-cycloalkyl, -CH=N-($C_1$-$C_{18}$alkyl),

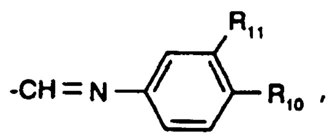

imidazolyl, pyrazolyl, triazolyl, piperazinyl, pyrrolyl, oxazolyl, benzoxazolyl, benzothiazolyl, benzimidazolyl, morpholinyl, piperidinyl or pyrrolidinyl,
G is -$CH_2$-, -CH($CH_3$)-, -C($CH_3$)$_2$-, -CH=N-, -N=N-, -O-, -S-, - SO-, -$SO_2$-, -CONH- or -$NR_7$-,
$R_{10}$ and $R_{11}$ are each independently of the other hydrogen, halogen, $C_1$-$C_6$alkyl, $C_1$-$C_{18}$alkoxy or -CN, and $R_{12}$ and $R_{13}$ are each independently of the other hydrogen, halogen or $C_1$-$C_6$alkyl.

**2.** A process for the preparation of a diketopyrrolopyrrole I according to claim 1, which comprises reacting a DPP derivative of formula III

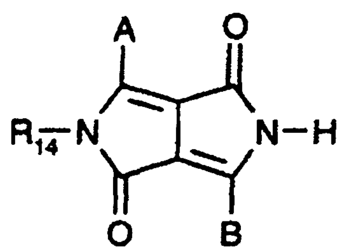

III,

wherein $R_{14}$ is hydrogen or $R_2$,
with a halogenated compound of formula IV

Hal-Y-X-Z-Q    IV

wherein Hal, Y, X, Z and Q are as defined in claim 1 in the presence of a base.

**3.** The use of a diketopyrrolopyrrole I according to claim 1 or prepared according to claim 2 for the preparation of a polymer.

**4.** A polymer based on a diketopyrrolopyrrole I according to claim 1, obtainable by polymerising at least one diketopyrrolopyrrole I according to claim 1 or a mixture consisting of

(A) from 0.5 to 20, preferably from 1 to 10 % by weight, based on the sum of the components (A) and (B), of a mixture consisting of

(a) from 100 to 1, preferably from 95 to 20, particularly preferably from 90 to 40 % by weight, based on the sum of the components (a) and (b), of a diketopyrrolopyrrole I,
(b) from to 0 to 99, preferably from 5 to 80, particularly preferably from 10 to 60 % by weight, based on the sum of the components (a) and (b), of the diketopyrrolopyrrole of formula V

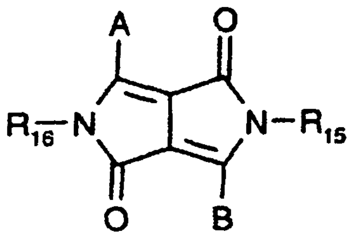

V,

wherein A and B are as defined in claim 1,
$R_{15}$ is a radical containing a polymerisable group, and
$R_{16}$ is $C_1$-$C_6$alkyl,

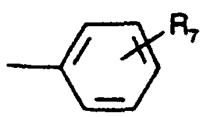

or $R_{15}$, and
(B) from 99.5 to 80, preferably from 99 to 90 % by weight, based on the sum of the components (A) and (B), of a monomer which is copolymerisable with the diketopyrrolopyrroles I and V,

the sums of (A) and (B) and of (a) and (b) each making up 100 % by weight.

**5.** A process for the preparation of a polymer according to claim 4, which comprises polymerising at least one diketopyrrolopyrrole I according to claim 1 or a mixture consisting of

(A) from 0.5 to 20, preferably from 1 to 10 % by weight, based on the sum of the components (A) and (B), of a mixture consisting of

(a) from 100 to 1, preferably from 95 to 20, particularly preferably from 90 to 40 % by weight, based on the sum of the components (a) and (b), of a diketopyrrolopyrrole I,
(b) from 0 to 99, preferably from 5 to 80, particularly preferably from 10 to 60 % by weight, based on the sum of the components (a) and (b), of the diketopyrrolopyrrole of formula V according to claim 4, and

(B) from 99.5 to 80, preferably from 99 to 90 % by weight, based on the sum of the components (A) and (B), of a monomer which is copolymerisable with the diketopyrrolopyrroles I and V,

the sums of (A) and (B) and of (a) and (b) each making up 100% by weight.

**6.** The use of a polymer according to claim 4 or prepared according to claim 5 for colouring high molecular weight organic materials, for formulations in decorative cosmetics, for the preparation of inks, including printing inks, coating materials, in particular automotive paints and photoresists, photo- and electroconducting polymers, fluorescent brighteners, photocell aggregates, coloured photoresists and emulsion paints.

**7.** A coloured high molecular weight organic material or coating material obtainable by the use of claim 6.

**8.** A mixture consisting of

(A) from 0.5 to 20, preferably from 1 to 10 % by weight, based on the sum of the components (A) and (B), of a mixture consisting of

(a) from 100 to 1, preferably from 95 to 20, particularly preferably from 90 to 40 % by weight, based on the sum of the components (a) and (b), of a diketopyrrolopyrrole I,
(b) from 0 to 99, preferably from 5 to 80, particularly preferably from 10 to 60 % by weight, based on the sum of the components (a) and (b), of the diketopyrrolopyrrole of formula V according to claim 4, and

(B) from 99.5 to 80, preferably from 99 to 90 % by weight, based on the sum of the components (A) and (B), of a monomer which is copolymerisable with the diketopyrrolopyrroles I and V,

the sums of (A) and (B) and of (a) and (b) each making up 100 % by weight.

**9.** The use of a mixture according to claim 8 for the preparation of a polymer.

**10.** The diketopyrrolopyrrole of formula

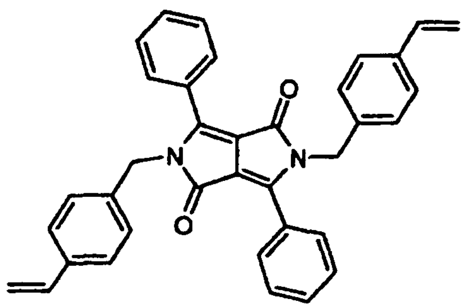

**Revendications**

**1.** Dicétopyrrolopyrroles de formule I

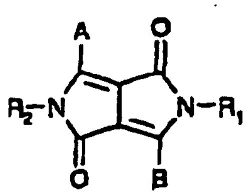

(I)

dans laquelle

$R_1$ représente un groupe de formule II

$$-Y-X-Z-Q \quad (II)$$

dans laquelle

Y représente $-CR_3R_4$, $-SO_2-$, -CO-, $-Si(Hal)_2-$, -POHal-, où $R_3$ et $R_4$ représentent, indépendamment l'un de l'autre, un atome d'hydrogène ou un groupe alkyle en $C_1$ à $C_4$ et Hal représente un atome d'halogène,
X représente un groupe phénylène, naphtylène, anthracénylène, anthraquinonylène, pyridinylène, quinolinylène ou phénylèneoxy,
Z représente une liaison simple, un groupe alkylène en $C_1$ à $C_{30}$ linéaire ou ramifié ininterrompu ou un groupe alkylène en $C_2$ à $C_{30}$ linéaire ou ramifié, interrompu une ou plusieurs fois par -O-, -S-, -CH=CH-, -C≡C-, -N=CH-, -CH=N- ou -NH-, ou un groupe cycloalkylène en $C_5$ à $C_{12}$,
Q représente un groupe -OH, -SH, $-NH_2$, glycidyle, 1,2-époxyéthyle,

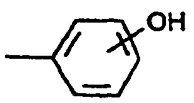 ,

-CHO, -NCO, $-CH=CH_2$, $-C(CH_3)=CH_2$, $-OCO-CH=CH_2$, $-OCO-C(Me)=CH_2$, $-CH_2-CH=CH_2$, $-CH_2-OH$, $-CO-CH=CH_2$, $-CO-C(CH_3)=CH_2$, cycloalcényle en $C_5$ à $C_7$,

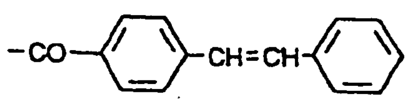 ,

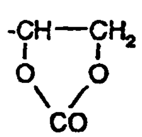 , 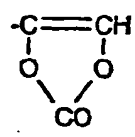 ,

$-CONHR_6$, $-COOR_6$, $-COR_6$, où $R_6$ représente un atome d'hydrogène ou un groupe alkyle en $C_1$ à $C_6$,

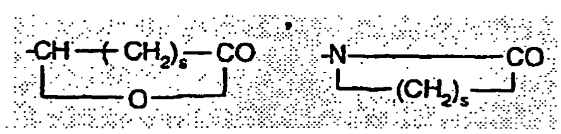

où s représente un nombre entier de 1 à 6, et
$R_2$ représente un groupe alkyle en $C_1$ à $C_6$,

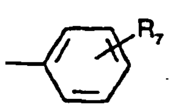

ou

$R_1$, et $R_7$ représente un atome d'hydrogène ou un groupe alkyle en $C_1$ à $C_6$,

et

A et B xreprésentent, indépendamment l'un de l'autre, un groupe de formule

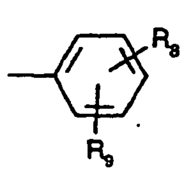, 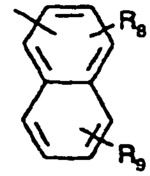, 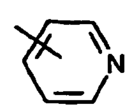, 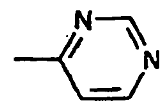,

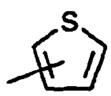, 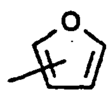 ou 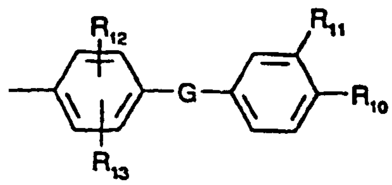

dans lesquelles

$R_8$ et $R_9$ représentent, indépendamment l'un de l'autre, un atome d'hydrogène, d'halogène, un groupe alkyle en $C_1$ à $C_{18}$, alcoxy en $C_1$ à $C_{18}$, alkylmercapto en $C_1$ à $C_{18}$, alkylamino en $C_1$ à $C_{18}$, alcoxy(en $C_1$ à $C_{18}$)carbonyle, alkyl(en $C_1$ à $C_{18}$)aminocarbonyle, -CN, $-NO_2$, trifluorométhyle, cycloalkyle en $C_5$ à $C_6$, -CH=N-(alkyle en $C_1$ à $C_{18}$),

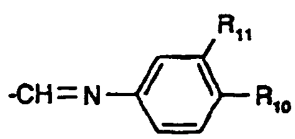,

imidazolyle, pyrrazolyle, triazolyle, pipérazinyle, pyrrolyle, oxazolyle, benzoxazolyle, benzothiazolyle, benzimidazolyle, morpholinyle, pipéridinyle ou pyrrolidinyle,

G représente $-CH_2-$, $-CH(CH_3)-$, $-C(CH_3)_2-$, -CH=N-, -N=N-, -O-, -S-, -SO-, $-SO_2-$, -CONH- ou $-NR_7-$,

$R_{10}$ et $R_{11}$ représentent, indépendamment l'un de l'autre, un atome d'hydrogène, d'halogène, un groupe alkyle en $C_1$ à $C_6$, alcoxy en $C_1$ à $C_{18}$ ou -CN,

$R_{12}$ et $R_{13}$ représentent, indépendamment l'un de l'autre, un atome d'hydrogène, d'halogène ou un groupe alkyle en $C_1$ à $C_6$.

2. Procédé de préparation des dicétopyrrolopyrroles I selon la revendication 1, **caractérisé en ce que** l'on fait réagir des dérivés de DPP de formule III

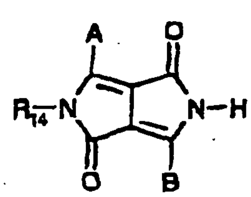

III

dans laquelle $R_{14}$ représente un atome d'hydrogène ou $R_2$, avec des composés halogénés de formule IV

$$Hal\text{-}Y\text{-}X\text{-}Z\text{-}Q \qquad IV$$

dans laquelle Hal, Y, X, Z et Q ont la signification indiquée dans la revendication 1, en présence d'une base.

**3.** Utilisation des dicétopyrrolopyrroles I selon la revendication 1 ou préparés selon la revendication 2 pour la production de polymères.

**4.** Polymères à base des dicétopyrrolopyrroles I selon la revendication 1, pouvant être obtenus par polyréaction d'au moins un dicétopyrrolopyrrole I selon la revendication 1 ou d'un mélange composé de

(A) 0,5% à 20%, de préférence 1% à 10% en poids, par rapport à la somme des composants (A) et (B), d'un mélange composé de

(a) 100% à 1%, de préférence 95% à 20%, de manière plus particulièrement préférée 90% à 40% en poids, par rapport à la somme des composants (a) et (b), d'un dicétopyrrolopyrrole I,
(b) 0% à 99%, de préférence 5% à 80%, de manière plus particulièrement préférée 10% à 60% en poids, par rapport à la somme des composants (a) et (b), du dicétopyrrolopyrrole de formule V,

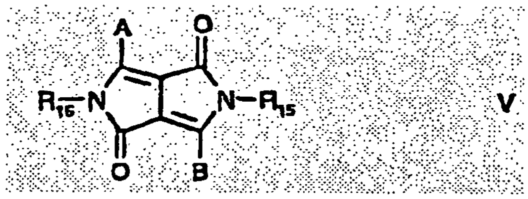
V

dans laquelle A et B ont la même signification que dans la revendication 1,
$R_{15}$ représente un groupe contenant un radical actif dans une polyréaction et
$R_{16}$ représente un groupe alkyle en $C_1$ à $C_6$,

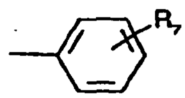

ou $R_{15}$,

(B) 99,5% à 80%, de préférence 99% à 90% en poids, par rapport à la somme des composants (A) et (B), d'un monomère copolymérisable avec les dicétopyrrolopyrroles I et V,

les sommes de (A) et (B) ainsi que de (a) et (b) étant respectivement égales à 100% en poids.

**5.** Procédé de production des polymères selon la revendication 4, **caractérisé en ce que** l'on soumet à une polyréaction au moins un dicétopyrrolopyrrole I selon la revendication 1 ou un mélange composé de

(A) 0,5% à 20%, de préférence 1% à 10% en poids, par rapport à la somme des composants (A) et (B), d'un mélange composé de

(a) 100% à 1%, de préférence 95% à 20%, de manière plus particulièrement préférée 90% à 40% en poids, par rapport à la somme des composants (a) et (b), d'un dicétopyrrolopyrrole I,
(b) 0% à 99%, de préférence 5% à 80%, de manière plus particulièrement préférée 10% à 60% en poids, par rapport à la somme des composants (a) et (b), du dicétopyrrolopyrrole de formule V selon la revendication 4, et

(B) 99,5% à 80%, de préférence 99% à 90% en poids, par rapport à la somme des composants (A) et (B), d'un monomère copolymérisable avec les dicétopyrrolopyrroles I et V,

les sommes de (A) et (B) ainsi que de (a) et (b) étant respectivement égales à 100% en poids.

**6.** Utilisation des polymères selon la revendication 4 ou préparés selon la revendication 5 pour colorer des matières organiques à haute masse moléculaire, pour des préparations de cosmétique décorative, pour fabriquer des encres, des encres d'imprimerie, des vernis, en particulier des vernis pour automobiles et des résines photosensibles, des polymères photoconducteurs et électroconducteurs, des azurants, des groupes de cellules photo-électriques, des photorésists teintés et des peintures en dispersion.

**7.** Matières organiques à haute masse moléculaire colorées et vernis pouvant être obtenus grâce à l'utilisation selon la revendication 6.

**8.** Mélange composé de

(A) 0,5% à 20%, de préférence 1% à 10% en poids, par rapport à la somme des composants (A) et (B), d'un mélange composé de

(a) 100% à 1%, de préférence 95% à 20%, de manière plus particulièrement préférée 90% à 40% en poids, par rapport à la somme des composants (a) et (b), d'un dicétopyrrolopyrrole I,
(b) 0% à 99%, de préférence 5% à 80%, de manière plus particulièrement préférée 10% à 60% en poids, par rapport à la somme des composants (a) et (b), du dicétopyrrolopyrrole de formule V selon la revendication 4 et

(B) 99,5% à 80%, de préférence 99% à 90% en poids, par rapport à la somme des composants (A) et (B), d'un monomère copolymérisable avec les dicétopyrrolopyrroles I et V, les sommes de (A) et (B) ainsi que de (a) et (b) étant respectivement égales à 100% en poids.

**9.** Utilisation du mélange selon la revendication 8 pour la production de polymères.

**10.** Dicétopyrrolopyrrole de formule